# EUROPEAN PATENT APPLICATION

(11) **EP 4 497 422 A1**
(43) Date of publication of application: **29.01.2025**
(21) Application number: 23807902.4
(22) Date of filing: 17.05.2023
(51) Int. Cl.: A61F 9/02, G02B 7/28, G02B 27/01, A61B 3/103

(54) **METHOD AND DEVICE FOR CORRECTING VISION ON BASIS OF FOCUS ADJUSTMENT LENS**

(30) Priority: 18.05.2022 KR 20220061029
(71) Applicant: Samsung Electronics Co., Ltd., Suwon-si, Gyeonggi-do 16677 (KR)
(72) Inventor: LEE, Kyookeun, Suwon-si, Gyeonggi-do 16677 (KR); MILTON, Harry Edward, Suwon-si, Gyeonggi-do 16677 (KR)
(74) Representative: Appleyard Lees IP LLP
(86) International application number: PCT/KR2023/006670
(87) International publication number: WO 2023/224383

(57) **Abstract**

A method of correcting vision by a vision correcting apparatus including a focus adjustable lens, includes: determining a first dioptric power to match far focus, based on far vision of a user; determining a second dioptric power to match near focus, based on corrected near vision of the user measured based on the first dioptric power; determining at least one third dioptric power to be applied to the focus adjustable lens, based on the first dioptric power and the second dioptric power; and controlling the focus adjustable lens based on the at least one third dioptric power.

## Description

### TECHNICAL FIELD

The disclosure relates to a method and apparatus for correcting the vision of a user, and more particularly, to a method and apparatus for correcting the far vision and near vision of a user by using a focus adjustable lens configured to adjust a focus by changing dioptric power (or refractive power).

### BACKGROUND ART

About 30% or more of the population may feel inconvenience in looking at an object with naked-eye vision due to an eye-lens refraction error or a ciliary accommodation error, and a pair of glasses for adjusting the refraction of light incident on the eyes may be necessary for vision correction.

An electronic focus adjustable lens may implement a lens with various focal powers by adjusting the dioptric power of a lens by using a voltage. Such a focus adjustable lens with a variable dioptric power may be universally applicable to various users and may correct the user's vision by applying a suitable dioptric power to the user even when the user's vision changes with time.

Augmented reality may be a technology for displaying an image by projecting a virtual image onto a real-world object or a physical environment space of the real world. An augmented reality device may display a real scene and a virtual image together through a glasses-type device using a see-through display such as a light guide plate (or a waveguide), which is arranged in front of the user's eyes while being worn on the user's face or head. As research has been actively conducted on such an augmented reality device, various types of wearable devices have been released or announced to be released. In the case of a wearable augmented reality device including a focus adjustable lens, it may correct the user's vision and may also measure the user's vision by using a display.

### DESCRIPTION OF EMBODIMENTS

### TECHNICAL PROBLEM

When the user is myopic, the user measures far vision and uses a pair of glasses with lenses suitable for the far vision. When the user uses a pair of glasses with lenses suitable for the far vision, the user's far vision may be corrected but the user's near focus may be distorted. For example, when the myopia of the far vision is overcorrected or when the user is presbyopic, an additional lens (or dioptric power or focal power) may be required to match the near focus.

### SOLUTION TO PROBLEM

According to an aspect of the disclosure, a method of correcting vision by a vision correcting apparatus including a focus adjustable lens, includes: determining a first dioptric power to match far focus, based on far vision of a user; determining a second dioptric power to match near focus, based on corrected near vision of the user measured based on the first dioptric power; determining at least one third dioptric power to be applied to the focus adjustable lens, based on the first dioptric power and the second dioptric power; and controlling the focus adjustable lens based on the at least one third dioptric power.

The determining the at least one third dioptric power may include: determining whether the user is presbyopic; based on determining that the user is not presbyopic, determining a dioptric power with overcorrection of the first dioptric power removed from the dioptric power based on the second dioptric power as the at least one third dioptric power; and based on determining that the user is presbyopic, determining a fourth dioptric power to correct near vision of the user and the first dioptric power as the at least one third dioptric power.

The method may further include: measuring the far vision of the user; and measuring the corrected near vision of the user based on the first dioptric power.

The corrected near vision of the user may be near vision of the user measured based on the focus adjustable lens with the first dioptric power applied to the focus adjustable lens.

The controlling the focus adjustable lens based on the at least one third dioptric power may include: controlling a first area of the focus adjustable lens based on the first dioptric power; and controlling a second area of the focus adjustable lens based on the fourth dioptric power.

The controlling the focus adjustable lens based on the at least one third dioptric power may include: identifying a distance between the vision correcting apparatus and an object; determining whether the distance between the vision correcting apparatus and the object is less than a threshold distance; based on determining that the distance between the vision correcting apparatus and the object is less than the threshold distance, controlling the focus adjustable lens based on the first dioptric power; and based on determining that the distance between the vision correcting apparatus and the object is greater than or equal to the threshold distance, controlling the focus adjustable lens based on the fourth dioptric power.

The controlling the focus adjustable lens based on the at least one third dioptric power may further include obtaining eye gaze information of the user, and the identifying the distance between the vision correcting apparatus and the object may include identifying the distance between the vision correcting apparatus and the object based on the eye gaze information of the user.

According to an aspect of the disclosure, an apparatus for correcting vision, includes: a focus adjustable lens; a storage storing a program including at least one instruction; and at least one processor configured to execute the at least one instruction to: determine a first dioptric power to match far focus, based on far vision of a user, determine a second dioptric power to match near focus based on corrected near vision of the user measured, based on the first dioptric power, determine at least one third dioptric power to be applied to the focus adjustable lens, based on the first dioptric power and the second dioptric power, and control the focus adjustable lens based on the at least one third dioptric power.

The at least one processor may be further configured to execute the at least one instruction to: determine whether the user is presbyopic, based on determining that the user is not presbyopic, determine a dioptric power with overcorrection of the first dioptric power removed from the dioptric power based on the second dioptric power as the at least one third dioptric power, and based on determining that the user is presbyopic, determine a fourth dioptric power to correct near vision of the user and the first dioptric power as the at least one third dioptric power.

The at least one processor may be further configured to execute the at least one instruction to: measure the far vision of the user; and measure the corrected near vision of the user based on the first dioptric power.

The corrected near vision of the user may be near vision of the user measured based on the focus adjustable lens with the first dioptric power applied to the focus adjustable lens.

The at least one processor may be further configured to execute the at least one instruction to control a first area of the focus adjustable lens based on the first dioptric power and control a second area of the focus adjustable lens based on the fourth dioptric power.

The at least one processor may be further configured to execute the at least one instruction to: identify a distance between a vision correcting apparatus and an object; determine whether the distance between the vision correcting apparatus and the object is less than a threshold distance; based on determining that the distance between the vision correcting apparatus and the object is less than the threshold distance, control the focus adjustable lens based on the first dioptric power, and based on determining that the distance between the vision correcting apparatus and the object is greater than or equal to the threshold distance, control the focus adjustable lens based on the fourth dioptric power.

The at least one processor may be further configured to execute the at least one instruction to: obtain eye gaze information of the user; and identify the distance between the vision correcting apparatus and the object based on the eye gaze information of the user.

According to an aspect of the disclosure, a non-transitory computerreadable recording medium has recorded thereon a program that is executable by a processor of a vision correcting apparatus to perform the method.

### BRIEF DESCRIPTION OF DRAWINGS

The above and other aspects, features, and advantages of example embodiments of the disclosure will be more apparent from the following description taken in conjunction with the accompanying drawings, in which:
FIG. 1 is a diagram illustrating an example of a vision correcting apparatus according to an embodiment of the disclosure;
FIG. 2 is a block diagram of a vision correcting apparatus according to an embodiment of the disclosure;
FIG. 3A illustrates a structure of a human eyeball;
FIG. 3B is a diagram illustrating a case where an emmetropic image is formed;
FIG. 4A illustrates a change in an eye lens depending on the distance between an object and the human eye;
FIG. 4B illustrates a change in an eye lens depending on the distance between an object and the human eye;
FIG. 5A illustrates an image formed when there is a refraction error due to myopia and a method of correcting the refraction error;
FIG. 5B illustrates an image formed when there is a refraction error due to myopia and a method of correcting the refraction error;
FIG. 5C illustrates an image formed when there is a refraction error due to myopia and a method of correcting the refraction error;
FIG. 5D illustrates an image formed when there is a refraction error due to myopia and a method of correcting the refraction error;
FIG. 6A illustrates an image formed when there is a refraction error due to hyperopia and a method of correcting the refraction error;
FIG. 6B illustrates an image formed when there is a refraction error due to hyperopia and a method of correcting the refraction error;
FIG. 6C illustrates an image formed when there is a refraction error due to hyperopia and a method of correcting the refraction error;
FIG. 6D illustrates an image formed when there is a refraction error due to hyperopia and a method of correcting the refraction error;
FIG. 7A illustrates an image formed when there is a refraction error due to astigmatism and a method of correcting the refraction error;
FIG. 7B illustrates an image formed when there is a refraction error due to astigmatism and a method of correcting the refraction error;
FIG. 7C illustrates an image formed when there is a refraction error due to astigmatism and a method of correcting the refraction error;
FIG. 7D illustrates an image formed when there is a refraction error due to astigmatism and a method of correcting the refraction error;
FIG. 8A illustrates an electrode arrangement of a focus adjustable lens in a vision correcting method according to an embodiment of the disclosure;
FIG. 8B illustrates an electrode arrangement of a focus adjustable lens in a vision correcting method according to an embodiment of the disclosure;
FIG. 8C is a perspective view illustrating a focus adjustable lens according to an embodiment of the disclosure;
FIG. 8D is a perspective view illustrating an operation of adjusting the dioptric power of a focal area of a focus adjustable lens by a vision correcting apparatus according to an embodiment of the disclosure;
FIG. 8E is a diagram for describing the concept of vergence of a focus adjustable lens that is a component of a vision correcting apparatus according to an embodiment of the disclosure;
FIG. 8F is a diagram for describing the concept of vergence of a focus adjustable lens that is a component of a vision correcting apparatus according to an embodiment of the disclosure;
FIG. 9 is a flowchart illustrating a vision correcting method according to an embodiment of the disclosure;
FIG. 10A illustrates a method of measuring far vision in a vision correcting method according to an embodiment of the disclosure;
FIG. 10B illustrates a method of measuring near vision in a vision correcting method according to an embodiment of the disclosure;
FIG. 11A is a diagram for describing a method of measuring near vision by using a vision correcting apparatus in a vision correcting method according to an embodiment of the disclosure;
FIG. 11B is a diagram for describing a method of measuring near vision by using a vision correcting apparatus in a vision correcting method according to an embodiment of the disclosure;
FIG. 12 is a flowchart illustrating a vision correcting method according to an embodiment of the disclosure;
FIG. 13 is a flowchart illustrating a vision correcting method according to an embodiment of the disclosure;
FIG. 14 is a flowchart illustrating a vision correcting method according to an embodiment of the disclosure; and
FIG. 15 is a flowchart illustrating a vision correcting method according to an embodiment of the disclosure.

### MODE OF DISCLOSURE

Throughout the disclosure, the expression "at least one of a, b or c" indicates only a, only b, only c, both a and b, both a and c, both b and c, or all of a, b, and c.

Hereinafter, an embodiment of the disclosure will be described in detail with reference to the accompanying drawings so that those of ordinary skill in the art may easily implement the embodiment of the disclosure. However, the disclosure may be embodied in many different forms and should not be construed as being limited to the embodiment set forth herein. Also, portions irrelevant to the description of the disclosure will be omitted in the drawings for a clear description of the disclosure, and like reference numerals will denote like elements throughout the specification.

Throughout the specification, when an element is referred to as being "connected" to another element, it may be "directly connected" to the other element or may be "electrically connected" to the other element with one or more intervening elements therebetween. Also, when something is referred to as "including" or "comprising" a component, unless there is a particular description contrary thereto, another component may be further included, not excluding the other component.

Herein, "augmented reality (AR)" may refer to displaying a virtual image in a physical environment space of the real world or displaying a real-world object and a virtual image together.

Also, an "augmented reality device" may be a device capable of representing "augmented reality" and may generally include not only glasses-type augmented reality glasses worn on the face of the user but also a head mounted display apparatus (HMD) or an augmented reality helmet worn on the head of the user.

Moreover, a "real scene" may be a scene of the real world that the user sees through the augmented reality device and may include a real-world object. Also, a "virtual image" may be an image generated through an optical engine and may include both a static image and a dynamic image. The virtual image may be displayed on a transparent display to be observed together with a real scene by a user and may be an image representing information about a real-world object in the real scene, information about an operation of the augmented reality device, a control menu, or the like.

Thus, a general augmented reality device may include an optical engine for generating a virtual image including light generated from a light source, and a waveguide through which the virtual image generated by the optical engine is guided to the user's eyes and is formed of a transparent material such that a scene of the real world may also be viewed together. As described above, because the augmented reality device should also let the user to observe a scene of the real world together with the virtual image, it may basically require an optical element for changing the path of light having straightness in order to guide the light generated by the optical engine to the user's eyes through the waveguide. **In** this case, the light path may be changed by using reflection by a mirror or the like, or the light path may be changed through diffraction by a diffraction element such as a diffractive optical element (DOE) or a holographic optical element (HOE); however, the disclosure is not limited thereto.

Hereinafter, the disclosure will be described in detail with reference to the accompanying drawings.

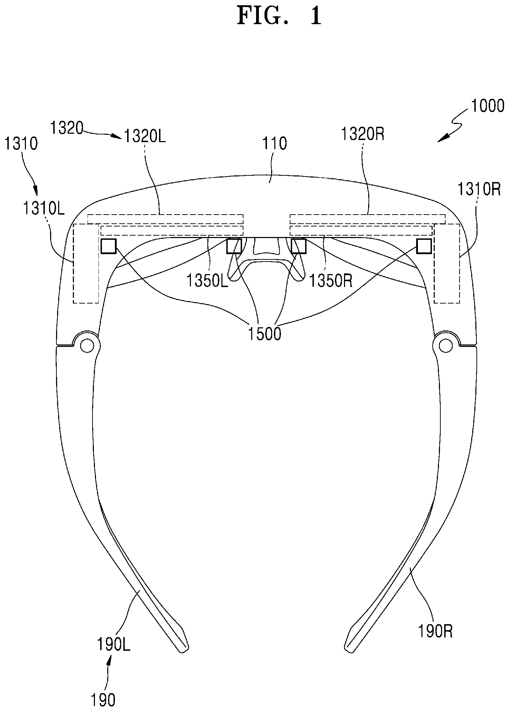

FIG. 1 is a diagram illustrating an example of a vision correcting apparatus according to an embodiment of the disclosure.

A vision correcting apparatus 1000 according to an embodiment of the disclosure may be, but is not limited to, an augmented reality device, may include a focus adjustable lens configured to adjust a focus, and may include any device that performs a function of correcting vision by using a focus adjustable lens.

Referring to FIG. 1, the vision correcting apparatus 1000 may be a glasses-type display apparatus and may include a glasses-type body configured to be worn by the user.

The glasses-type body may include a frame (or rim) 110 and a support unit 190, and the support unit 190 may extend from the frame 110 and may be used to seat the augmented reality device on the user's head. The support unit 190 may include a temple 190L and 190R and a nose support unit. The temple 190L and 190R may extend from the frame 110 and may be used to fix the vision correcting apparatus 1000 to the user's head at the side of the glasses-type body. The nose support unit may extend from the frame 110 and may be used to seat the vision correcting apparatus 1000 on the user's nose and may include, for example, a nose bridge and a glasses nose;. However, embodiments are not limited thereto.

Also, a lens unit 1350 and a waveguide 1320 may be located in the frame 110. The lens unit 1350 may include a left-eye lens unit 1350L and a right-eye lens unit 1350R. Also, the waveguide 1320 may be configured, in which an input of projected light is received into an input area and at least a portion of the input light is output from an output area. The waveguide 1320 may include a left-eye waveguide 1320L and a right-eye waveguide 1320R.

The lens unit 1350 may include a focus adjustable lens. Unlike a related lens in which the focal length or dioptric power of the lens is fixed, the focus adjustable lens may be a lens that may change in the shape or dioptric power of the lens by an external stimulus or device setting, and may be arranged in parallel to the waveguide 1320. The unit of dioptric power D may be diopter, and the dioptric power may be defined as the reciprocal of a focal length (m).

When a control voltage is applied to the focus adjustable lens, an arrangement angle of liquid crystal molecules arranged in a focal area of the focus adjustable lens may be changed and accordingly the dioptric power of the focal area may be adjusted. By executing program code or instructions related to dioptric power adjustment, a processor of the vision correcting apparatus 1000 may adjust the dioptric power of the focal area and may adjust the refractive index of light passing through the focal area. The processor of the vision correcting apparatus 1000 may adjust the vergence of a focus adjustable lens 1350 by adjusting the dioptric power of the focal area.

The "vergence" may be an index representing the degree of convergence or divergence of light and may be adjusted according to the dioptric power of the lens. In an embodiment of the disclosure, by adjusting the dioptric power of the focal area in a first direction, the processor of the vision correcting apparatus 1000 may adjust the vergence of the focus adjustable lens 1350 and may adjust a focal length for a real-world object or a virtual image. When the vergence of the focal area is adjusted in a divergence direction, the path of light passing through the focal area may increase and thus a focal length for a real-world object or a virtual image formed on the retinas of the user's eyes may increase.

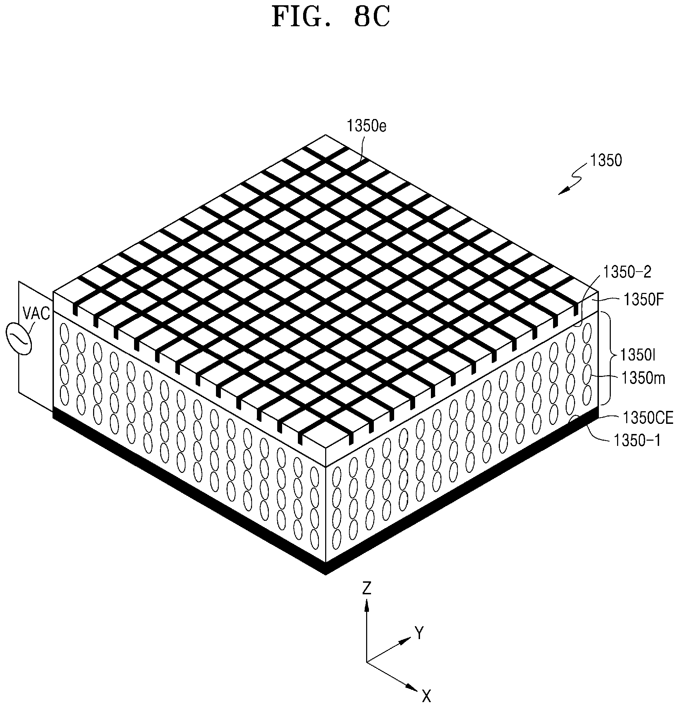

The processor of the vision correcting apparatus 1000 may correct the vision of the user's eyes by adjusting the dioptric power of the focal area and adjusting the focal length thereof. In this case, the focus adjustable lens 1350 may be used as a vision correcting lens. An embodiment in which the processor adjusts the dioptric power of the focal area and changes the vergence of the focal area will be described in detail with reference to FIGS. 8C to 8F.

The left-eye lens unit 1350L and the left-eye waveguide 1320L may be arranged at a position corresponding to the user's left eye, and the right-eye lens unit 1350R and right-eye waveguide 1320R may be arranged at a position corresponding to the user's right eye. For example, the left-eye lens unit 1350L and the left-eye waveguide 1320L may be attached to each other, or the right-eye lens unit 1350R and the right-eye waveguide 1320R may be attached to each other. However, embodiments are not limited thereto.

Also, an optical engine 1310 of a projector projecting the display light containing an image may include a left-eye optical engine 1310L and a right-eye optical engine 1310R. The left-eye optical engine 1310L and the right-eye optical engine 1310R may be located on both sides of the vision correcting apparatus 1000. According to another embodiment, one optical engine 1310 may be included in a center portion around the nose support unit of the vision correcting apparatus 1000. The light emitted from the optical engine 1310 may be displayed through the waveguide 1320.

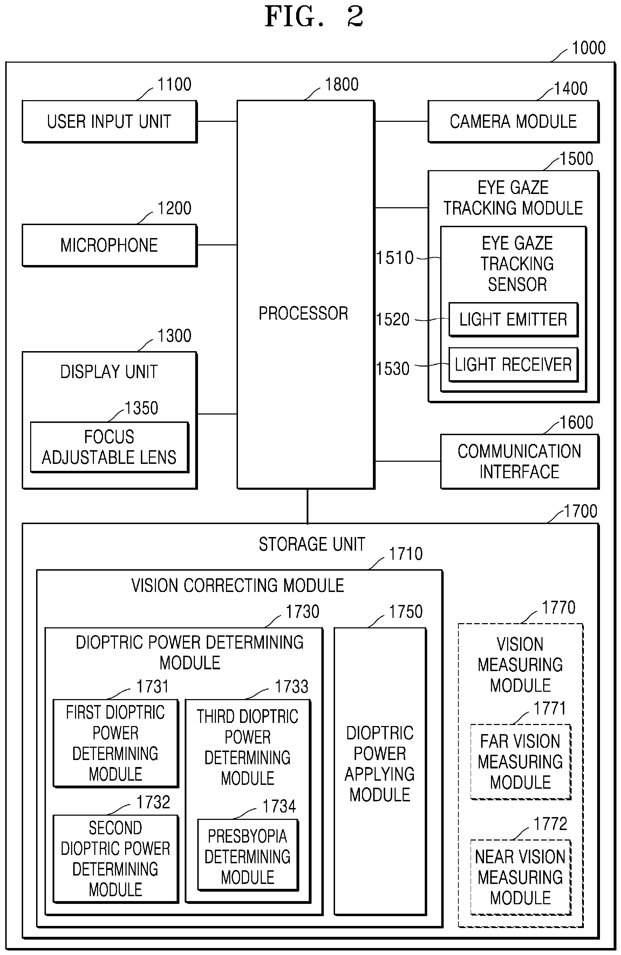

FIG. 2 is a block diagram of a vision correcting apparatus according to an embodiment of the disclosure.

A vision correcting apparatus 1000 according to an embodiment of the disclosure may be, but is not limited to, an augmented reality device, may include a focus adjustable lens, and may include any device that performs a function of correcting vision by using a focus adjustable lens.

Referring to FIG. 2, the vision correcting apparatus 1000 according to an embodiment of the disclosuremay include a user input unit 1100, a microphone 1200, a display unit 1300, a camera module 1400, an eye gaze detecting module 1500, a communication interface 1600, a storage unit 1700, a processor 1800, a speaker, and a depth sensor. Also, the display unit 1300 may include a focus adjustable lens 1350.

The user input unit 1100 may refer to a unit through which the user inputs data for controlling the vision correcting apparatus 1000. For example, the user input unit 1100 may include, but is not limited to, at least one of a key pad, a dome switch, a touch pad (e.g., a capacitive overlay type, a resistive overlay type, an infrared beam type, a surface acoustic wave type, an integral strain gauge type, or a piezoelectric type), a jog wheel, or a jog switch.

The microphone 1200 may receive an external audio signal and process the same into electrical voice data. For example, the microphone 1200 may receive an audio signal from an external device or a speaker. The microphone 1200 may use various noise removal algorithms for removing the noise generated in the process of receiving the external audio signal. The microphone 1200 may receive a user's voice input for controlling the vision correcting apparatus 1000.

The display unit 1300 may display and output information processed by the vision correcting apparatus 1000. For example, the display unit 1300 may display information related to a service provided based on an image obtained around the vision correcting apparatus 1000 and a user interface for photographing around the vision correcting apparatus 1000.

According to an embodiment of the disclosure, the display unit 1300 may provide an augmented reality (AR) image. As in FIG. 1, the display unit 1300 may include an optical engine 1310 and a waveguide 1320. The waveguide 1320 may include a transparent material through which a partial area of the rear surface is visible when the user wears the vision correcting apparatus 1000. The waveguide 1320 may include a flat plate having a single-layer or multilayer structure of a transparent material in which light may be reflected and transmitted. The waveguide 1320 may face an output surface of the optical engine 1310 to receive the light of a virtual image projected from the optical engine 1310. Here, the transparent material may refer to a material through which light may pass, may not have a transparency of 100 %, and may have a certain color. In an embodiment of the disclosure, as the waveguide 1320 includes a transparent material, because the user may not only see a virtual object of a virtual image through the display unit 1300 but also see an external real scene, the waveguide 1320 may be referred to as a see-through display. The display unit 1300 may provide an AR image by outputting a virtual object of a virtual image through the waveguide 1320. When the vision correcting apparatus 1000 is a glasses-type device, the display unit 1300 may include a left display unit and a right display unit.

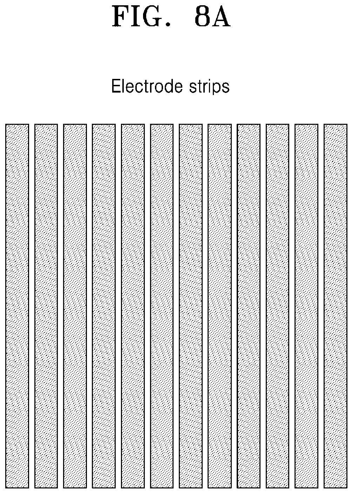

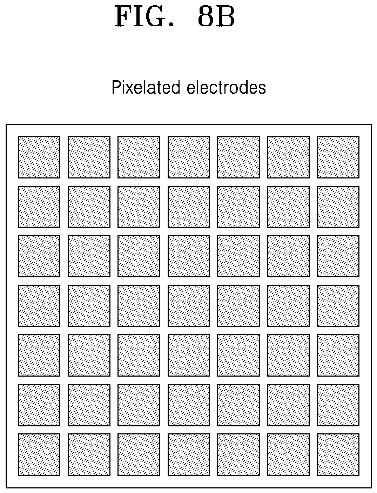

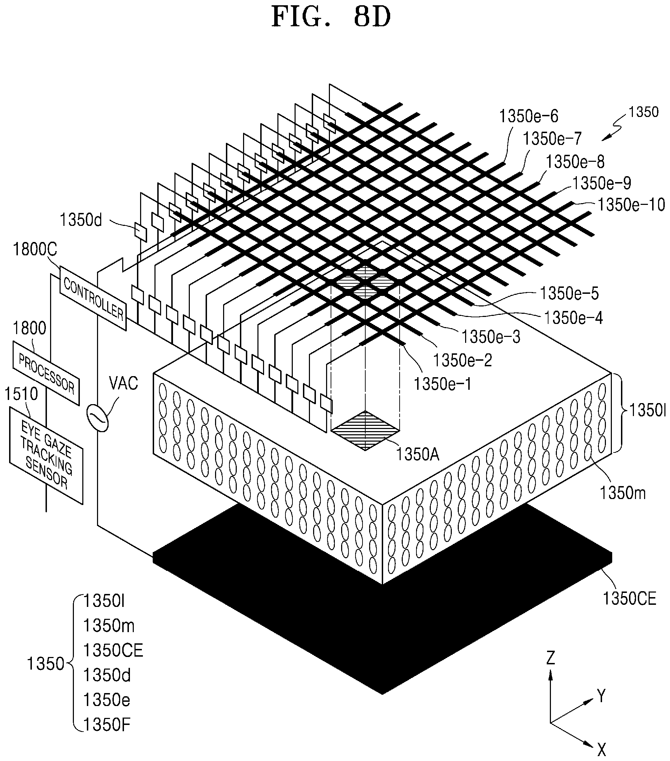

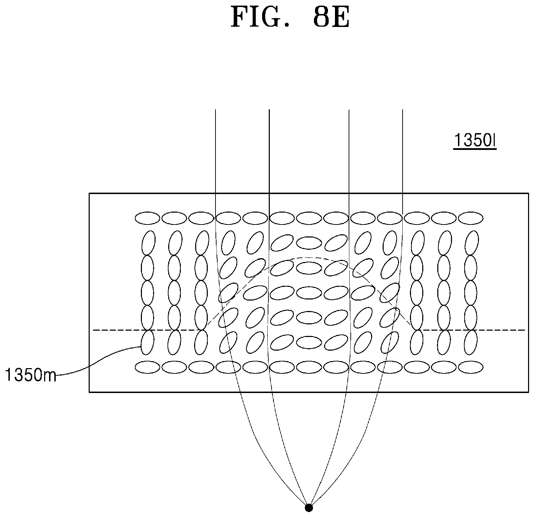

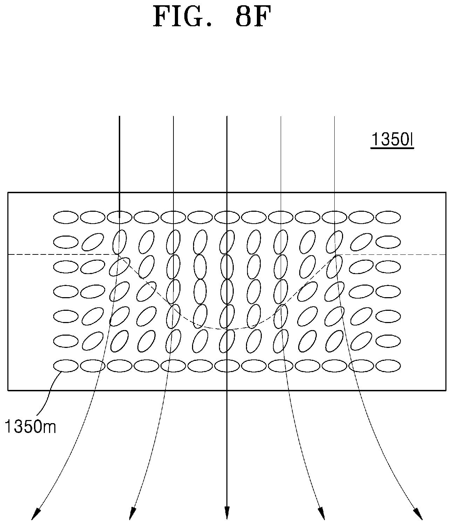

According to an embodiment of the disclosure, the display unit 1300 may include a focus adjustable lens 1350 as in FIG. 1. The focus adjustable lens 1350 may adjust the focal length of an AR image provided through the waveguide 1320 or an image viewed by the user through the display unit formed of a transparent material. Similar to the waveguide 1320, the focus adjustable lens 1350 may also include a transparent material through which a partial area of the rear surface is visible when the user wears the vision correcting apparatus 1000. Because the permittivity of the focus adjustable lens 1350 may be changed by an electrical input or an external stimulus applied to the focus adjustable lens 1350 and the dioptric power of the focus adjustable lens 1350 may be changed when the permittivity thereof is changed, the vision correcting apparatus 1000 may adjust the dioptric power of the focus adjustable lens 1350 by adjusting an electrical input or an external stimulus applied to the focus adjustable lens 1350. A method of determining the dioptric power of the focus adjustable lens 1350 by the vision correcting apparatus 1000 according to an embodiment of the disclosure will be described below with reference to FIGS. 8 to 15.

The focus adjustable lens 1350 may be a lens configured to change a focus according to an electrical driving signal. The focus adjustable lens 1350 may include a focal area configured to change or adjusting a focus. In an embodiment of the disclosure, the focus adjustable lens 1350 may include liquid crystal molecules and may include an electrically tunable liquid crystal lens configured to change a focus according to an electrical driving signal. In this case, the focus adjustable lens 1350 may locally change the dioptric power by changing the arrangement angle of liquid crystal molecules arranged in a particular area (e.g., the focal area) according to a control voltage applied from a battery. The position of an area where the focus is changed, that is, the focal area, may be moved on the focus adjustable lens 1350. The control voltage may be controlled by the processor 1800 and applied to the focus adjustable lens 1350 by a voltage control circuit. An embodiment in which dioptric power of the focal area is changed by application of the control voltage will be described in detail with reference to FIGS. 8C and 8D.

When the user wears the vision correcting apparatus 1000, the focus adjustable lens 1350 may include a left-eye varifocal lens 1350L (see FIG. 1) arranged in an area corresponding to the user's left eye and a right-eye varifocal lens 1350R (see FIG. 1) arranged in an area corresponding to the user's right eye. Each of the left-eye varifocal lens 1350L and the right-eye varifocal lens 1350R may include a single lens. However, embodiments are not limited thereto. In an embodiment of the disclosure, the left-eye varifocal lens 1350L may include a plurality of left-eye varifocal lenses and the right-eye varifocal lens 1350R may include a plurality of right-eye varifocal lenses.

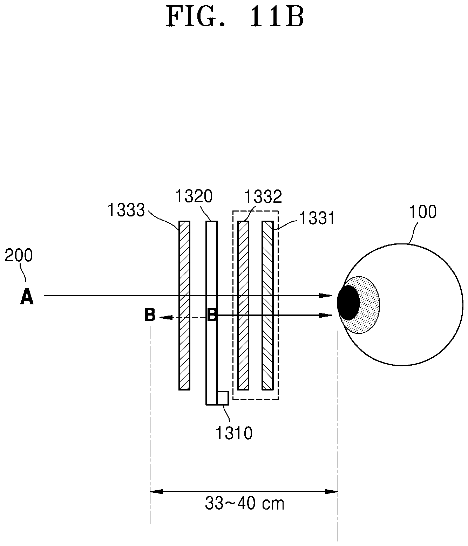

According to an embodiment of the disclosure, the display unit 1300 may measure near vision based on a virtual image projected on the waveguide 1320 by using a plurality of focus adjustable lenses 1350. A method of measuring near vision by the vision correcting apparatus 1000 according to an embodiment of the disclosure will be described below with reference to FIG. 11B.

The camera module 1400 may obtain an image around the vision correcting apparatus 1000. The camera module 1400 may obtain an image frame such as a still image or a moving image through an image sensor when an application requesting a photographing function is executed. The image obtained through the image sensor may be processed through the processor 1800 or a separate image processor. The camera module 1400 may include, for example, at least one of a rotatable RGB camera module or a plurality of depth camera modules. However, embodiments are not limited thereto.

The eye gaze detecting module 1500 may detect and track the eye gaze of the user wearing the vision correcting apparatus 1000. The eye gaze detecting module 1500 may be installed in a direction toward the user's eyes and may detect the eye gaze direction of the user's left eye and the eye gaze direction of the user's right eye. Detecting the user's eye gaze direction may include obtaining eye gaze information related to the user's eye gaze.

The eye gaze detecting module 1500 may include an eye gaze tracking sensor 1510 configured to emit infrared (IR) light and receiving IR light to detect the user's eye gaze. The eye gaze tracking sensor 1510 may include a light emitter 1520 emitting IR light and a light receiver 1530 receiving IR light and may obtain information related to the eyeball and eye gaze of the user wearing the vision correcting apparatus 1000.

The light emitter 1520 of the eye gaze detecting module 1500 may emit IR light such that IR light may be directed to the user's eyes. Also, the IR light reflected from the user's eyes may be received by the light receiver 1530 of the eye gaze detecting module 1500. The light receiver 1530 may be arranged at a position configured to receive the IR light reflected from the user's eyes in the vision correcting apparatus 1000.

The light emitter 1520 and the light receiver 1530 of the eye gaze detecting module 1500 may be arranged at a position on the inner surface of the support unit 190 between the support unit 190 and the user's eyes in the support unit 190 of the vision correcting apparatus 1000. In this case, the vision correcting apparatus 1000 may further include a light reflector, and the light emitter 1520 and the light receiver 1530 may be arranged to face the light reflector in the support unit 190 of the vision correcting apparatus 1000. The light emitter 1520 and the light receiver 1530 may be located at the support unit 190 of FIG. 1 supporting the vision correcting apparatus 1000 on the user's face, for example, at the temple and the nose support unit of FIG. 1.

The light reflector may reflect the light emitted from the light emitter 1520. The light reflector and the waveguide 1320 may be arranged at a position facing the user's eyes, and the light reflector and the waveguide 1320 may be attached to each other. The **IR** light emitted from the light emitter 1520 may be reflected by the light reflector and directed to the user's eyes, and the IR light reflected back from the user's eyes may be reflected by the light reflector and directed to the light receiver 1530.

The eye gaze detecting module 1500 may provide sensor data to the processor 1800, and the processor 1800 may obtain eye gaze information of the user based on the sensor data. The sensor data may be data obtained by the eye gaze tracking sensor 1510 of the eye gaze detecting module 1500 and may include the type of IR light emitted from the light emitter 1520 (e.g., point light, linear light, or surface light), the characteristics of IR light emitted from the light emitter 1520, data about an emission area of IR light emitted from the light emitter 1520, and data representing the characteristics of IR light received from the light receiver 1530.

Also, the eye gaze information of the user may be information related to the user's eye gaze, may be generated by analyzing the sensor data, and may include, for example, information about the position of the user's pupil, the position of the center point of the pupil, the position of the user's iris, the center of the user's eye, the position of the user's eye glint feature point, the user's gaze point, and the user's eye gaze direction. However, embodiments are not limited thereto. The user's eye gaze direction may be, for example, the direction of the eye gaze from the center of the user's eye toward the gaze point at which the user gazes. For example, the user's eye gaze direction may be represented by the value of a vector from the center of the user's left eye toward the gaze point and the value of a vector from the center of the user's right eye toward the gaze point. However, embodiments are not limited thereto.

According to an embodiment of the disclosure, the eye gaze detecting module 1500 may detect sensor data including information related to the eyeball and eye gaze of the user wearing the vision correcting apparatus 1000 at predetermined time intervals.

The communication interface 1600 may transmit/receive data for receiving a service related to the vision correcting apparatus 1000, to/from an external device and a server.

The storage unit 1700 may store a program to be executed by the processor 1800 described below and may store data input to or output from the vision correcting apparatus 1000.

The storage unit 1700 may include at least one of an internal memory or an external memory. The internal memory may include, for example, at least one of a volatile memory (e.g., dynamic RAM (DRAM), static RAM (SRAM), synchronous dynamic RAM (SDRAM), or the like), a non-volatile memory (e.g., one-time programmable ROM (OTPROM), programmable ROM (PROM), erasable and programmable ROM (EPROM), electrically erasable and programmable ROM (EEPROM), mask ROM, flash ROM, or the like), a hard disk drive (HDD), or a solid state drive (SSD). According to an embodiment of the disclosure, the processor 1800 may load a command or data received from at least one of the non-volatile memory or other components into the volatile memory and process the same. Also, the processor 1800 may store data received or generated from other components in a non-volatile memory. The external memory may include, for example, at least one of Compact Flash (CF), Secure Digital (SD), Micro Secure Digital (Micro-SD), Mini Secure Digital (Mini-SD), extreme Digital (xD), or Memory Stick.

Programs stored in the storage unit 1700 may be classified into a plurality of modules according to their functions and may include, for example, a vision correcting module 1710, a dioptric power determining module 1730, and a dioptric power applying module 1750. For example, a memory may be included in the dioptric power determining module 1730, and in this case, a first dioptric power determining module 1731, a second dioptric power determining module 1732, and a third dioptric power determining module 1733 may be stored as firmware in the memory included in the dioptric power determining module 1730.

The processor 1800 may control an overall operation of the vision correcting apparatus 1000. For example, the processor 1800 may overall control the user input unit 1100, the microphone 1200, the display unit 1300, the camera module 1400, the eye gaze detecting module 1500, the communication interface 1600, and the storage unit 1700 by executing the programs stored in the storage unit 1700.

The processor 1800 may correct the user's vision by executing the vision correcting module 1710, the dioptric power determining module 1730, and the dioptric power applying module 1750 stored in the storage unit 1700.

According to an embodiment of the disclosure, the vision correcting apparatus 1000 may include a plurality of processors 1800, and the vision correcting module 1710, the dioptric power determining module 1730, and the dioptric power applying module 1750 may be executed by the plurality of processors 1800.

For example, some of the vision correcting module 1710, the dioptric power determining module 1730, and the dioptric power applying module 1750 may be executed by a first processor, and the others of the vision correcting module 1710, the dioptric power determining module 1730, and the dioptric power applying module 1750 may be executed by a second processor. However, embodiments are not limited thereto.

The processor 1800 may determine a dioptric power to be applied to the focus adjustable lens 1350 by executing the dioptric power determining module 1730 and may control the focus adjustable lens 1350 by executing the dioptric power applying module 1750, and the focus adjustable lens 1350 controlled by the processor 1800 may correct the user's vision by refracting incident light.

The vision correcting module 1710 may include a first dioptric power determining module 1731, a second dioptric power determining module 1732, and a third dioptric power determining module 1733, and the third dioptric power determining module 1733 may include a presbyopia determining module 1734.

The first dioptric power determining module 1731 may determine a first dioptric power for far focus matching based on the user's far vision. When the user is myopic, because the far focus of light passing through the eye lens is formed before the retina, the first dioptric power may have a negative value and the magnitude of the first dioptric power may vary according to the degree of myopia of the user. Because a lens having a negative dioptric power (e.g., a concave lens) spreads the light incident on the lens, when a negative dioptric power is applied to the focus adjustable lens 1350, the far focal length of light passing through the eye lens may increase and thus a far image may be formed on the retina.

The second dioptric power determining module 1732 may determine a second dioptric power to match near focus based on the user's corrected near vision measured based on the first dioptric power.

Because the light passing from a distance through the focus adjustable lens with the first dioptric power applied thereto forms an image on the retina, the user viewing a far object through the focus adjustable lens with the first dioptric power applied thereto may more accurately recognize an image of the far object. However, when the user is presbyopic or the first dioptric power is excessively determined compared to the degree of myopia of the user, the focus adjustable lens with the first dioptric power applied thereto may not match the focus of light reflected from a near object and incident on the eye to the retina. Thus, the user's corrected near vision measured based on the focus adjustable lens 1350 with the first dioptric power applied thereto may require an additional dioptric power adjustment, that is, the second dioptric power, to match near focus. In this case, in the case of presbyopia or myopia overcorrection, because the near focus of light passing through the eye lens is formed behind the retina, the second dioptric power may have a positive value compared to the first dioptric power and the magnitude of the second dioptric power may vary according to the degree of presbyopia of the user or the degree of myopia overcorrection. Because a lens having a positive dioptric power (e.g., a convex lens) concentrates the light incident on the lens, when a positive dioptric power is applied to the focus adjustable lens 1350, the near focal length of light passing through the eye lens may decrease and thus a near image may be formed on the retina.

The third dioptric power determining module 1733 may determine a third dioptric power to be applied to the focus adjustable lens 1350, based on the first dioptric power and the second dioptric power.

The presbyopia determining module 1734 may determine whether the user is presbyopic, based on the user's age or the second dioptric power.

The vision correcting apparatus 1000 may ask the user's age by using an output unit such as the display unit 1300 or the speaker (not illustrated) and may obtain the user's age information by using an input unit such as the user input unit 1100, the microphone 1200, or the eye gaze detecting module 1500. Because presbyopia occurs with the increase of age and the occurrence rate thereof reaches about 100 %, it may be assumed that the user is presbyopic when the user is over a certain threshold age. However, the certain threshold age may vary according to races, regions, or other living environments. Also, because the second dioptric power generally has a greater value in the case of presbyopia, it may be assumed that the user is presbyopic when the magnitude of the second dioptric power is greater than a certain threshold value.

When it is determined that the user is not presbyopic, the third dioptric power determining module 1733 may remove the overcorrection from the first dioptric power based on the second dioptric power. According to an embodiment of the disclosure, the third dioptric power may be determined as the sum of the first dioptric power and the second dioptric power. In this case, a dioptric power for correcting the user's myopia may be determined as the third dioptric power.

When it is determined that the user is presbyopic, the third dioptric power determining module 1733 may determine a fourth dioptric power to match near focus based on the second dioptric power and a residual accommodative power of the user's eyes. When a separate dioptric power to match near focus is prescribed because the user is presbyopic, the third dioptric power may include a plurality of dioptric powers including the first dioptric power to match far focus and the fourth dioptric power to match near focus.

The dioptric power applying module 1750 may control the focus adjustable lens 1350 based on the third dioptric power.

The dioptric power of the focus adjustable lens 1350 may be changed by an external stimulus or device setting, and particularly, in the case of a liquid crystal (LC) lens, the dioptric power may be adjusted by adjusting a voltage applied to each electrode according to an electrode arrangement for an LC element. Thus, the dioptric power applying module 1750 may determine a voltage applied to the focus adjustable lens 1350 and control the focus adjustable lens 1350 by applying the determined voltage to the focus adjustable lens 1350 through the processor 1800. The electrode arrangement of the LC lens will be described below with reference to FIGS. 8A and 8B.

According to an embodiment of the disclosure, when it is determined that the user is presbyopic, the dioptric power applying module 1750 may differently control the dioptric power of the focus adjustable lens 1350 in a case where the user views a far object and in a case where the user views a near object, based on the output of the depth sensor or the eye gaze detecting module 1500, in order to control a variable lens according to the dioptric power for far vision correction and the dioptric power for near vision correction.

According to an embodiment of the disclosure, when it is determined that the user is presbyopic, the dioptric power applying module 1750 may control the focus adjustable lens 1350 such that a dioptric power for far vision correction may be applied to a first area and a dioptric power for near vision correction may be applied to a second area, by distinguishing between the areas of the focus adjustable lens 1350.

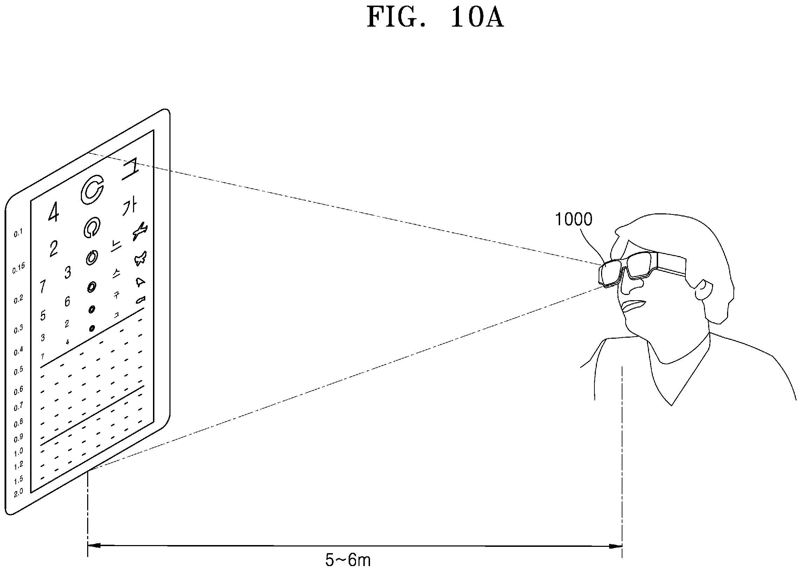

According to an embodiment of the disclosure, the storage unit 1700 may further include a vision measuring module 1770, and the vision measuring module 1770 may include a far vision measuring module 1771 and a near vision measuring module 1772. When the storage unit 1700 includes the vision measuring module 1770, a vision measurement table (or a virtual vision chart) for vision measurement may be displayed on the waveguide 1320 of the display unit 1300 and far vision measurement and near vision measurement may be performed based on the output of the eye gaze detecting module 1500 or the user's response. A method of measuring the near vision and a method of measuring the far vision will be described below with reference to FIGS. 10A to 11B.

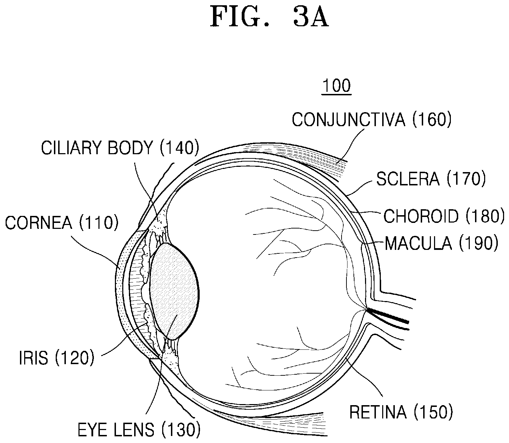

FIG. 3A illustrates a structure of a human eyeball.

An eyeball 100 of the human may be a visual organ transmitting visual information to the brain through a multi-level structure and may uniformly adjust the amount of light entering the eyes, focus on a distance where an object is located, and generate a continuous image that is immediately transmitted to the brain.

Referring to FIG. 3A, the eyeball 100 may include various structures such as a cornea 110, an iris 120, an eye lens 130, a ciliary body 140, a retina 150, a conjunctiva 160, a sclera 170, a choroid 180, and a macula 190.

The cornea 110 may be a portion where the sclera 170 extends toward the front of the eye and is located on the outermost surface of the eyeball and may refer to a portion covering the black part of the eye. The cornea 110 may function as a window of the eye and may protect the eye from the outside, pass light, and refract passed light.

The iris 120 may be a donut-shaped membrane around the pupil and may be located between the cornea 110 and the eye lens 130 and may adjust the amount of light entering the eyeball 100 by adjusting the size of the pupil through contraction and relaxation.

The eye lens 130 may refer to a transparent tissue in the form of a biconvex lens that is located behind the iris 120 and before a vitreous body in the eyeball 100. When light passes therethrough, the thickness and curvature of the eye lens 130 may be adjusted to concentrate and focus the light such that an image may be formed on the retina 150. The top and bottom of the eye lens 130 may be connected to a ciliary muscle by a ciliary zonule that is a thin fiber.

The ciliary body 140 may be located between the choroid 180 and the iris 120 and may surround the eye lens 130. The ciliary body 140 may fix the eye lens 130 by the ciliary zonule and simultaneously may adjust the focus by changing the curvature of the eye lens 130 by the ciliary muscle. When the ciliary muscle contracts, the ciliary zonule fixing the eye lens 130 may be stretched and the eye lens 130 may be thickened to focus on a near object, and when the ciliary muscle relaxes, the ciliary zonule may be shortened and the eye lens 130 may be thinned to focus on a far object.

The retina 150 may be a transparent nerve tissue located at the rear of the eyeball and covering about 2/3 of the inside of the eyeball and may refer to an innermost portion of the eyeball wall. The retina 150 may include nerve cells and neuroglia cells, and a fovea thereof, in which many cones that are color sensing cells are gathered and which is slightly concave, may have the highest resolution.

The fovea may be a portion at which light is formed after passing perpendicularly through the centers of the cornea and the eye lens, and the macula 190 that is a yellow portion around the fovea may be the center of a visual field.

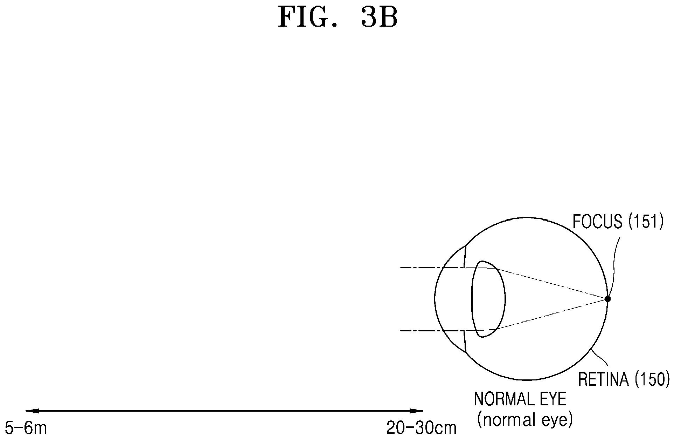

FIG. 3B is a diagram illustrating a case where an emmetropic image is formed.

Light entering from a distance greater than about 5 m to about 6 m may be assumed to enter in parallel to the eye. Parallel rays incident on the eye may be refracted by the cornea and the eye lens, an accurate image may be recognized when the refracted rays meet at the retina to form an image, and a case where the refracted rays meet at the retina to form an image may be referred to as emmetropia (or normal eye).

Referring to FIG. 3B, emmetropia may refer to a case where parallel rays incident on the eye after being reflected from an object located at a distance of about 5 m to about 6 m are refracted by the eye lens and thus a focus 151 is formed on the retina 150, that is, a case where an image is formed on the retina 150. In order to achieve emmetropia, the dioptric power of the cornea and the eye lens and an ocular axis (the front-to-back length of the eye) should match well.

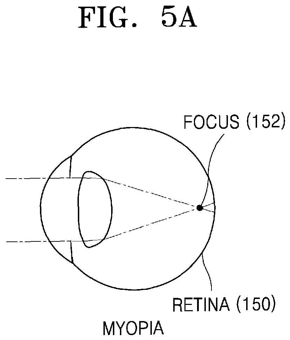

Among the cases where emmetropia is not achieved, for example, a case where a focus is formed before the cornea may be referred to as myopia, and a case where a focus is formed behind the cornea may be referred to as hyperopia. A method of correcting the relationship between the eyeball and the image in the case of myopia or hyperopia will be described below with reference to FIGS. 5A to 6D.

It is known that the dioptric power of the cornea is an average of 51.2D at birth and is an average of 43.5D in adults, the dioptric power of the eye lens is an average of 34.3D at birth and is an average of 18.8D in adults, and the length of the ocular axis is an average of 16.8 mm at birth and is an average of 23.6 mm in adults. The human vision at birth is in a hyperopic state of about +2D to about 3D, and the length of the ocular axis increases greatly until the age of 3 after birth and reaches the adult length at about 14 years of age. With growth, the length of the ocular axis may increase and the curvature of the cornea and the eye lens may decrease and thus the eye may gradually become emmetropia, but ametropia due to a refraction error such as myopia, hyperopia, or astigmatism may occur according to habit or heredity.

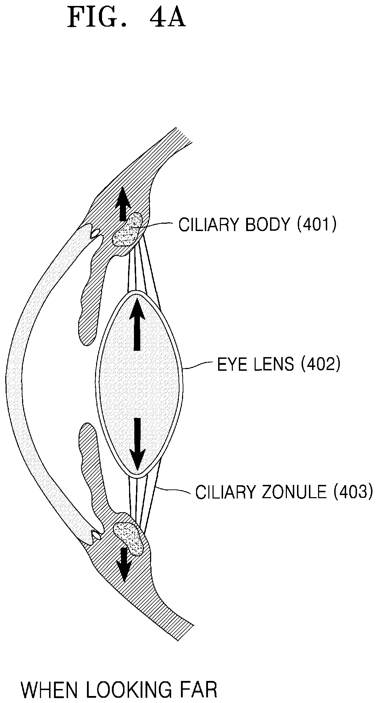

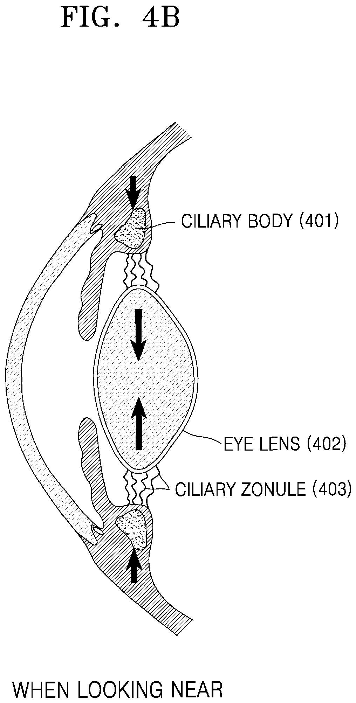

FIGS. 4A and 4B illustrate a change in an eye lens depending on the distance between an object and the human eye.

The human may clearly recognize an object only when light entering the eye is suitably refracted while passing through the cornea and the eye lens and thus an accurate focus is formed at the fovea.

The dioptric power of the cornea may be fixed at about 23°; however, because the thickness of the eye lens may be adjusted, the dioptric power of the eye lens may change from about 11° to about 18° in the case of young humans. The unit of dioptric power may be diopter D, may be commonly referred to as focal power, and may be determined as the reciprocal of an origin distance (m) or the reciprocal of a focal length (m) in an optical lens.

Referring to FIG. 4A, when looking at a far object, as a ciliary muscle 401 relaxes, a ciliary zonule 403 may be shortened and thus an eye lens 402 may be thinned. When the eye lens 402 is thinned, it may focus on the far object.

Referring to FIG. 4B, when looking at a near object, as the ciliary muscle 401 contracts, the ciliary zonule 403 may be stretched and thus the eye lens 402 may be thickened. When the eye lens 402 is thickened, it may focus on the near object.

With the increase of age, the elasticity of the eye lens 402 may decrease and an accommodative power for changing the dioptric power may decrease and thus presbyopia due to an accommodation error may occur. Presbyopia may be diagnosed when a near point, which is the closest distance that may be clearly seen when the accommodation is maximized, is less than 25 cm, that is, when the accommodative power is greater than or equal to 4D. In this case, when an object at up to 25 cm in front may be clearly seen, the accommodative power may be 1/0.25m=4D, and when an object at up to 10 cm in front may be clearly seen, the accommodative power may be 1/0.1m=10D.

FIGS. 5A to 5D illustrate an image formed when there is a refraction error due to myopia and a method of correcting the refraction error.

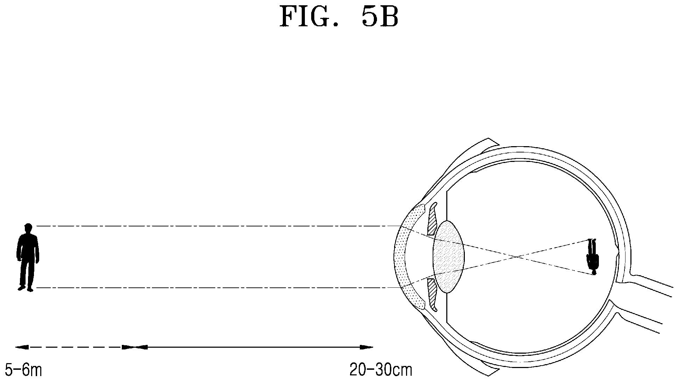

FIGS. 5A and 5B are diagrams illustrating that a focus and an image of myopia are formed.

As described above, light entering from a distance greater than about 5 m to about 6 m may be assumed to enter in parallel to the eye. Parallel rays incident in parallel to the eye may be refracted by the cornea and the eye lens, and an accurate image may be recognized when the refracted rays meet at the retina to form an image. In the case of myopia, refraction may occur excessively or the length of the ocular axis may increase and thus an image may be formed before the retina, and a myopic person may unclearly see or may not well see a far object. However, even when the person is myopic, the person may normally see a near object. That is, a near focus may be formed on the retina.

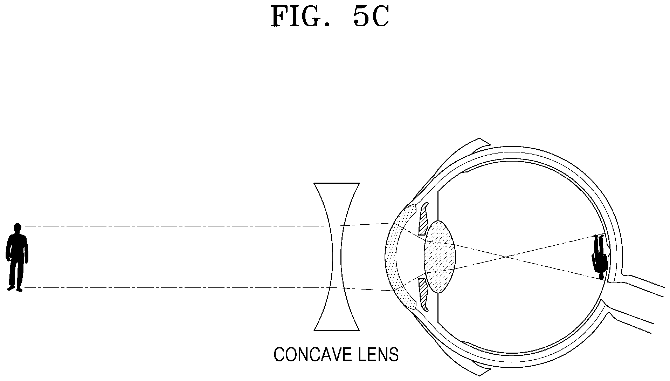

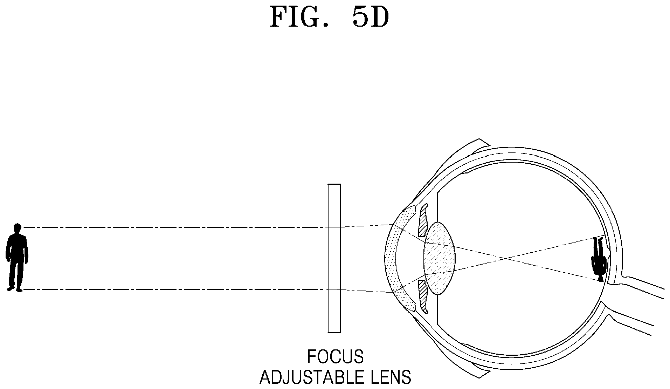

FIGS. 5C and 5D are diagrams illustrating a method of correcting myopia by using a lens.

As described above, in the case of myopia, an image may be formed before the retina due to excessive refraction or a long ocular axis. Because the length of the ocular axis may not be artificially adjusted, when a concave lens is used to refract the light incident on the lens to diverge outward as illustrated in FIG. 5C in order to offset excessive refraction in the eye, an image may be formed on the retina even when the light entering the eye is strongly refracted by the eye lens.

According to an embodiment of the disclosure, the vision correcting apparatus 1000 may perform the same function by using the focus adjustable lens 1350 instead of a concave lens. Because the dioptric power of the focus adjustable lens 1350 may be adjusted according to an external stimulus or an applied voltage, the light incident on the lens may be refracted to spread outward by adjusting the voltage of the focus adjustable lens 1350 such that the dioptric power of the focus adjustable lens 1350 may be less than the dioptric power of air.

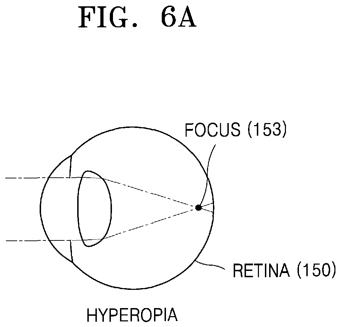

FIGS. 6A to 6D illustrate an image formed when there is a refraction error due to hyperopia and a method of correcting the refraction error.

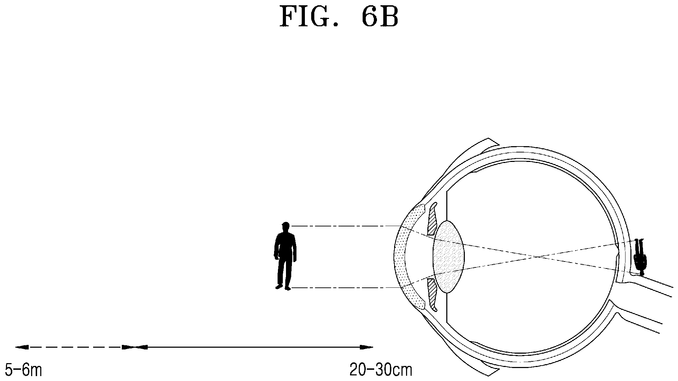

FIGS. 6A and 6B are diagrams illustrating that a focus and an image of hyperopia are formed.

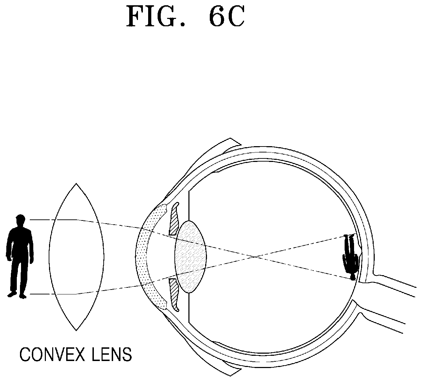

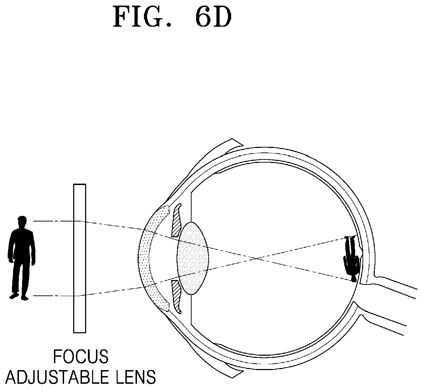

In the case of hyperopia, refraction may be weak or the length of the ocular axis may be small and thus an image may be formed behind the retina, and a hyperopic person may unclearly see or may not well see a near object. However, even when the person is hyperopic, the person may normally see a far object. That is, a far focus may be formed on the retina. FIGS. 6C and 6D are diagrams illustrating a method of correcting hyperopia by using a lens.

As described above, in the case of hyperopia, an image may be formed behind the retina due to weak refraction or a short ocular axis. Because the length of the ocular axis may not be artificially adjusted, when a convex lens is used to refract the light incident on the lens to converge inward as illustrated in FIG. 6C in order to further refract the light incident on the eye, an image may be formed on the retina even when the light entering the eye is weakly refracted by the eye lens.

According to an embodiment of the disclosure, the vision correcting apparatus 1000 may perform the same function by using the focus adjustable lens 1350 instead of a convex lens. Because the dioptric power of the focus adjustable lens 1350 may be adjusted according to an external stimulus or an applied voltage, the light incident on the lens may be refracted to converge inward by adjusting the voltage of the focus adjustable lens 1350 such that the dioptric power of the focus adjustable lens 1350 may be greater than the dioptric power of air.

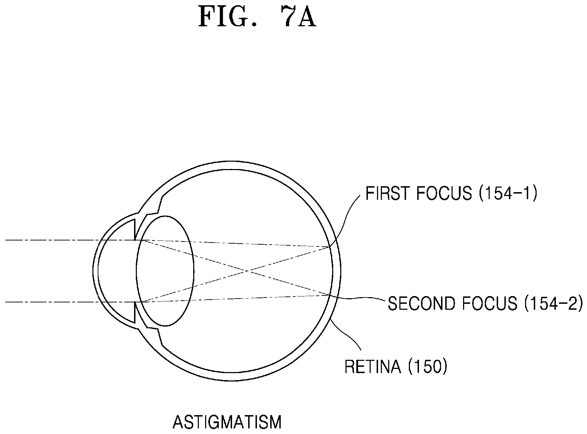

FIGS. 7A to 7D illustrate an image formed when there is a refraction error due to astigmatism and a method of correcting the refraction error.

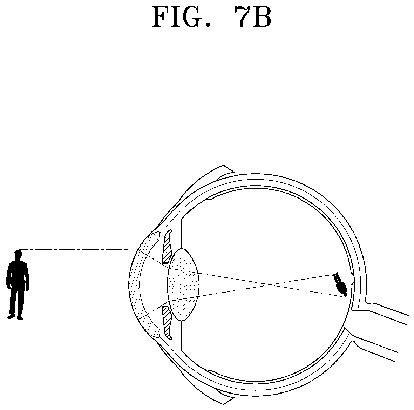

FIGS. 7A and 7B are diagrams illustrating that a focus and an image of astigmatism are formed.

Astigmatism and hyperopia or astigmatism and myopia may occur simultaneously, and in this case, an image may be formed behind or before the retina respectively. Herein, for convenience of description, it is assumed that only astigmatism occurs.

Unlike in emmetropia, hyperopia, or myopia in which a focus is formed at one point, in astigmatism, because the eye's dioptric power is not equal across all meridians, a focus may not be formed at one point but two or more focuses may be formed. That is, a refraction surface may not have a spherical shape but may have a distorted shape. Referring to FIG. 7B, an image formed by multiple focuses may be recognized in a distorted manner by the human.

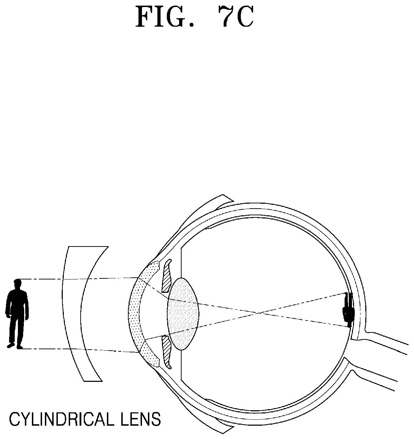

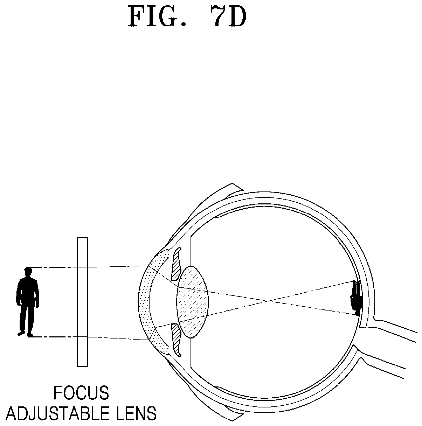

FIGS. 7C and 7D are diagrams illustrating a method of correcting astigmatism by using a lens.

As described above, because astigmatism occurs due to asymmetrical refraction, when a cylindrical lens is used to adjust refraction as illustrated in FIG. 7C in order to differently apply the refraction of light incident on each region of the eye, the light entering the eye may be refracted by the eye lens and thus a single focus may be formed.

According to an embodiment of the disclosure, the vision correcting apparatus 1000 may perform the same function by using the focus adjustable lens 1350 instead of a cylindrical lens. Because the dioptric power of the focus adjustable lens 1350 may be adjusted according to an external stimulus or an applied voltage, the light incident on the lens may be refracted to compensate for a refraction error of the eye lens by adjusting the voltage of the focus adjustable lens 1350 such that the dioptric power of each portion of the focus adjustable lens 1350 may be differently applied.

FIGS. 8A and 8B illustrate an electrode arrangement of a focus adjustable lens in a vision correcting method according to an embodiment of the disclosure.

According to an embodiment of the disclosure, the focus adjustable lens 1350 may be implemented by using a liquid crystal (LC) element, and the focus adjustable lens 1350 implemented by using the LC element will be referred to as an LC lens.

An electrode for driving the LC lens may be variously configured. Vision correction using the LC lens may refer to adjusting the dioptric power of the LC element like the dioptric power for vision correction by applying a suitable voltage to the electrode of the LC element.

Referring to FIG. 8A, a cylindrical focus adjustable lens according to an embodiment of the disclosuremay be implemented by using an LC lens including strip electrodes.

Because the dioptric power of a cylindrical lens may change in a horizontal, vertical, or diagonal direction, a cylindrical focus adjustable lens with dioptric power changing in each direction may be implemented by arranging strip electrodes in the horizontal, vertical, or diagonal direction of a focus adjustable lens and adjusting an applied voltage.

Referring to FIG. 8B, a focus adjustable lens according to an embodiment of the disclosuremay be implemented by using an LC lens including pixelated electrodes.

A gradient index (GRIN) spherical lens with dioptric power changing continuously according to positions may be implemented by applying different voltages to pixelated electrodes. According to an embodiment of the disclosure, a pixelated electrode may be formed by arranging two strip electrodes to intersect each other in directions orthogonal to each other (e.g., the horizontal direction and the vertical direction), and a lens may be moved in each orthogonal direction.

FIG. 8C is a perspective view illustrating a focus adjustable lens according to an embodiment of the disclosure.

Referring to FIG. 8C, a focus adjustable lens 1350 may include a liquid crystal layer 1350I, a common electrode 1350CE, a transparent film 1350F, and an excitation electrode 1350e. The focus adjustable lens 1350 may further include a transparent layer formed to contact the lower surface of the common electrode 1350CE.

The focus control lens 1350 may be an electrically tunable liquid crystal lens configured to adjust the refractive index of light by changing the arrangement angle of liquid crystal molecules 1350m based on a control voltage applied from a power supply unit VAC through the excitation electrode 1350e. In an embodiment of the disclosure, the focus adjustable lens 1350 may include an electro-optic material including a pixel grid. Pixels may be arranged in a matrix of N rows and M columns. Each of the N×M pixels may accommodate a set of possible values (gray levels) independent of all other pixels.

The liquid crystal layer 1350I may be an electro-optic layer including a plurality of liquid crystal molecules 1350m. The liquid crystal layer 1350I may be an electro-optic layer in which the physical properties of liquid crystal are changed by an applied control voltage. In an embodiment of the disclosure, the liquid crystal layer 1350I may include a polarization-independent liquid crystal layer (e.g., cholesteric liquid crystal). In the liquid crystal layer 1350I, the refractive index of a particular area may be locally adjusted by changing the arrangement angle of the liquid crystal molecules 1350m arranged in a particular area in an active area according to a control voltage applied through the excitation electrode 1350e.

The common electrode 1350CE and the excitation electrode 1350e may receive a control voltage from the power supply unit VAC and apply the received control voltage to the liquid crystal layer 1350I. The common electrode 1350CE may be arranged to contact a first surface 1350-1 of the liquid crystal layer 1350I.

The excitation electrode 1350e may be arranged over a second surface 1350-2 opposite to the first surface 1350-1 of the liquid crystal layer 1350I to contact the upper surface of the transparent film 1350F. The excitation electrode 1350e may include a first array excitation electrode and a second array excitation electrode oriented in orthogonal directions along the X-axis and Y-axis directions on the upper surface of the transparent film 1350F. Each of the first array excitation electrode and the second array excitation electrode may include parallel strips of conductive material extending over the active area. In an embodiment of the disclosure, the excitation electrode 1350e may include a transparent conductive material such as indium tin oxide (ITO).

A pixel may be defined by an area where the strip of the first array excitation electrode and the strip of the second array excitation electrode overlap each other. The center-to-center distance between the strip of the first array excitation electrode and the strap of the second array excitation electrode may define the pitch of a pixel array, and the width of the strip may define the size of the pixel.

The processor 1800 (see FIG. 2) of the vision correcting apparatus 1000 may apply a control voltage waveform having a phase modulation profile to the excitation electrode 1350e through the power supply unit VAC and may modulate the control voltage applied to the excitation electrode 1350e. As a control voltage having a waveform modulated by the processor 1800 is applied, the dioptric power of the focus adjustable lens 1350 may be locally adjusted in a particular area in the active area by the phase modulation profile of the applied control voltage. The focus adjustable lens 1350 may function as a vergence lens according to the adjusted dioptric power. Here, the vergence may be an index representing the degree of convergence or divergence of light and may be adjusted according to the dioptric power of the focus adjustable lens 1350. In an embodiment of the disclosure, the focus adjustable lens 1350 may adjust the vergence by changing the ray or light path by adjusting the dioptric power of the lens.

The processor 1800 may change the focal length by adjusting the vergence of a particular area of the focus adjustable lens 1350, that is, a focal area thereof. A particular method of the processor 1800 determining the position of a focal area 1350A (see FIG. 8D) of the focus adjustable lens 1350 and adjusting the dioptric power of the focal area 1350A will be described in detail with reference to FIG. 8D.

FIG. 8D is a perspective view illustrating an operation of adjusting the dioptric power of a focal area of a focus adjustable lens by a vision correcting apparatus according to an embodiment of the disclosure.

Referring to FIG. 8D, a focus adjustable lens 1350 may include a liquid crystal layer 1350I, liquid crystal molecules 1350m, a common electrode 1350CE, a plurality of driver terminals 1350d, a plurality of first array excitation electrodes 1350e-1 to 1350e-5, a plurality of second array excitation electrodes 1350e-6 to 1350e-10, and a transparent film 1350F. Unlike in FIG. 8C, for convenience of description, the transparent film 1350F is not illustrated in FIG. 8D.

The plurality of first array excitation electrodes 1350e-1 to 1350e-5 may be arranged in the X-axis direction, and the plurality of second array excitation electrodes 1350e-6 to 1350e-10 may be arranged in the Y-axis direction. The plurality of first array excitation electrodes 1350e-1 to 1350e-5 and the plurality of second array excitation electrodes 1350e-6 to 1350e-10 may be arranged to be orthogonal to each other.

A plurality of driver terminals 1350d for controlling the control voltage applied from the power supply unit VAC to the plurality of first array excitation electrodes 1350e-1 to 1350e-5 may be respectively connected to the plurality of first array excitation electrodes 1350e-1 to 1350e-5. A plurality of driver terminals 1350d for controlling the control voltage applied from the power supply unit VAC to the plurality of second array excitation electrodes 1350e-6 to 1350e-10 may be respectively connected to the plurality of second array excitation electrodes 1350e-6 to 1350e-10.

A controller 1800C may be electrically and/or physically connected to the plurality of driver terminals 1350d and the power supply unit VAC. In FIG. 8D, the controller 1800C is illustrated as a separate component from the processor 1800. However, embodiments are not limited thereto. In an embodiment of the disclosure, the controller 1800C and the processor 1800 may be integrated into a single component.

By controlling the plurality of driver terminals 1350d, the controller 1800C may control the control voltage applied to the plurality of first array excitation electrodes 1350e-1 to 1350e-5 and the plurality of second array excitation electrodes 1350e-6 to 1350e-10 and accordingly may adjust the arrangement angle of liquid crystal molecules arranged in a particular area. Unlike the illustration in 8D, in an embodiment of the disclosure, the focus adjustable lens 1350 may not include the plurality of driver terminals 1350d and the controller 1800C may be directly connected to the plurality of first array excitation electrodes 1350e-1 to 1350e-5 and the plurality of second array excitation electrodes 1350e-6 to 1350e-10.

The eye gaze tracking sensor 1510 may obtain an eye gaze vector by tracking the eye gaze direction of the user's eyes and may provide the obtained eye gaze vector to the processor 1800. The processor 1800 may calculate the position coordinate values of an area at which the eye gaze arrives among the entire area of the focus adjustable lens 1350 based on the vector direction of the eye gaze vector and may provide information about the calculated position coordinate values to the controller 1800C. The controller 1800C may determine a focal area 1350A, which is a target area for focus adjustment, based on the position coordinate values obtained from the processor 1800.

In the embodiment illustrated in FIG. 8D, in order to change the arrangement angle of liquid crystal molecules arranged in the focal area 1350A among the plurality of liquid crystal molecules 1350m included in the liquid crystal layer 1350I, a voltage may be controlled to be applied to a second excitation electrode 1350e-2, a third excitation electrode 1350e-3, and a fourth excitation electrode 1350e-4 among the plurality of first array excitation electrodes 1350e-1 to 1350e-5, and a voltage may be controlled to be applied to a seventh excitation electrode 1350e-7, an eighth excitation electrode 1350e-8, and a ninth excitation electrode 1350e-9 among the plurality of second array excitation electrodes 1350e-6 to 1350e-10. In an embodiment of the disclosure, by controlling the plurality of driver terminals 1350d, the controller 1800C may control the power supply unit VAC to apply a voltage to the second excitation electrode 1350e-2, the third excitation electrode 1350e-3, and the fourth excitation electrode 1350e-4 and to apply a voltage to the seventh excitation electrode 1350e-7, the eighth excitation electrode 1350e-8, and the ninth excitation electrode 1350e-9. In this case, the controller 1800C may control the plurality of driver terminals 1350d such that a voltage may not be applied to the first excitation electrode 1350e-1, the fifth excitation electrode 1350e-5, the sixth excitation electrode 1350e-6, and the tenth excitation electrode 1350e-10.

The controller 1800C not only may control whether to apply a control voltage from the power supply unit VAC but also may control the level of the control voltage applied from the power supply unit VAC. The controller 1800C may adjust the size of the alignment angle of the liquid crystal molecules by controlling the level of the applied control voltage. For example, when the controller 1800C applies a control voltage with a first level to the second excitation electrode 1350e-2 and applies a control voltage with a second level higher than the first level to the third excitation electrode 1350e-3 through the plurality of driver terminals 1350d, the alignment angle of liquid crystal molecules located in an area where the third excitation electrode 1350e-3 is arranged among the entire area of the liquid crystal layer 1350I may be adjusted to be greater than the arrangement angle of liquid crystal molecules located in an area where the second excitation electrode 1350e-2 is arranged.

That is, by modulating the phase profile of the control voltage applied to the plurality of first array excitation electrodes 1350e-1 to 1350e-5 and the plurality of second array excitation electrodes 1350e-6 to 1350e-10 through the plurality of driver terminals 1350d, the controller 1800C may determine the focal area 1350A in which the arrangement angle of the liquid crystal molecules 1350m among the entire area of the liquid crystal layer 1350I is changed and may adjust the dioptric power of the focal area 1350A.

FIGS. 8E and 8F are conceptual diagrams for describing the concept of vergence of a focus adjustable lens that is a component of a vision correcting apparatus according to an embodiment of the disclosure.

Referring to FIGS. 8E and 8F, when a control voltage modulated to have a particular phase profile is applied to the liquid crystal layer 1350I of the focus adjustable lens 1350, the alignment angle of the liquid crystal molecules 1350m arranged at a particular position in the active area may be changed. As the arrangement angle of the liquid crystal molecules 1350m arranged in a particular area of the liquid crystal layer 1350I is changed, the refractive index of light passing through the liquid crystal molecules 1350m may be changed. When the refractive index of light is changed, the dioptric power of the focus adjustable lens 1350 may be changed and thus the path of light passing through the focus adjustable lens 1350 may be changed and accordingly the vergence may be changed. The vergence may be an index representing the degree of convergence or divergence of light passing through the focus adjustable lens 1350. The vergence may be adjusted according to the dioptric power of the focus adjustable lens 1350.

In the embodiment illustrated in FIG. 8E, the light passing through an area in which the arrangement angle of the liquid crystal molecules 1350m included in the liquid crystal layer 1350I is changed may form a positive vergence and accordingly the focus adjustable lens 1350 may perform the same function as a convex lens. When the positive vergence is formed, the focal length may decrease.

In the embodiment illustrated in FIG. 8F, the light passing through an area in which the rotation angle of the liquid crystal molecules 1350m included in the liquid crystal layer 1350I is changed may form a negative vergence and accordingly the focus adjustable lens 1350 may perform the same function as a concave lens. When the negative vergence is formed, the focal length may increase.

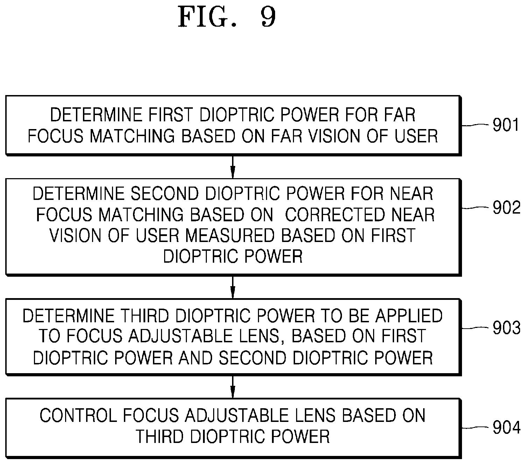

FIG. 9 is a flowchart illustrating a vision correcting method according to an embodiment of the disclosure.

Referring to FIG. 9, the vision correcting apparatus 1000 according to an embodiment of the disclosuremay correct the user's vision based on the user's far vision and near vision.

In operation S901, the vision correcting apparatus 1000 may determine a first dioptric power to match far focus based on the user's far vision.

When the user is myopic, a lens with a first dioptric power having a minus diopter may be prescribed based on the result of measurement of the user's far vision. According to an embodiment of the disclosure, when the user is myopic, because the far focus of light passing through the eye lens is formed before the retina, the first dioptric power may have a negative value and the magnitude of the first dioptric power may vary according to the degree of myopia of the user. Because a lens having a negative dioptric power (e.g., a concave lens) spreads the light incident on the lens, when a negative dioptric power is applied to the focus adjustable lens 1350, the far focal length of light passing through the eye lens may increase and thus a far image may be formed on the retina.

In operation S902, the vision correcting apparatus 1000 may determine a second dioptric power to match near focus based on the user's corrected near vision measured based on the first dioptric power.

When the vision is self-measured by using a subjective refraction test method without expert intervention, myopia overcorrection may occur. That is, as a result of the far vision measurement, a lens having a stronger focal power than an actually necessary focal power may be prescribed for correcting the user's myopia. Thus, the vision correcting apparatus 1000 according to an embodiment of the disclosuremay correct a dioptric power to be applied to the focus adjustable lens 1350, based on the user's corrected near vision corrected by the first dioptric power determined based on the far vision measurement result.

According to an embodiment of the disclosure, because the light passing from a distance through the focus adjustable lens with the first dioptric power applied thereto forms an image on the retina, the user viewing a far object through the focus adjustable lens with the first dioptric power applied thereto may accurately recognize an image of the far object. However, when the user is presbyopic or the first dioptric power is excessively determined compared to the degree of myopia of the user, the focus adjustable lens with the first dioptric power applied thereto may not match the focus of light reflected from a near object and incident on the eye to the retina. Thus, the user's corrected near vision measured based on the focus adjustable lens 1350 with the first dioptric power applied thereto may require an additional dioptric power adjustment, that is, the second dioptric power, to match near focus. **In** this case, in the case of presbyopia or myopia overcorrection, because the near focus of light passing through the eye lens is formed behind the retina, the difference between the second dioptric power and the first dioptric power may have a positive value and the magnitude of the second dioptric power may vary according to the degree of presbyopia of the user or the degree of myopia overcorrection. Because a lens having a positive dioptric power (e.g., a convex lens) concentrates the light incident on the lens, when a positive dioptric power is applied to the focus adjustable lens 1350, the near focal length of light passing through the eye lens may decrease and thus a near image may be formed on the retina.

**In** operation S903, the vision correcting apparatus 1000 may determine a third dioptric power to be applied to the focus adjustable lens, based on the first dioptric power and the second dioptric power.

According to an embodiment of the disclosure, when it is determined that the user is not presbyopic, the third dioptric power may be determined as the sum of the first dioptric power and the second dioptric power. **In** this case, a dioptric power for correcting the user's myopia may be determined as the third dioptric power.

According to an embodiment of the disclosure, when it is determined that the user is presbyopic, the third dioptric power may include a plurality of dioptric powers including the first dioptric power to match far focus and a fourth dioptric power to match near focus, and the fourth dioptric power may refer to a dioptric power to match near focus that is determined based on the residual accommodative power of the user's eyes and the second dioptric power.

A vision measuring and correcting method according to an embodiment of the disclosuremay include predicting whether the user is presbyopic or the degree of presbyopia based on age information of the user and correcting myopia overcorrection based on the far vision measurement result based on the prediction result.

For example, when the user is 33 years of age, it may be predicted based on the age of the user that the user is not presbyopic. It is assumed that a lens having a dioptric power of left eye -2.25D SPH, right eye -1.25D SPH, and -0.50D CYL@90° is prescribed for a corrected vision of 1.0 as a result of the far vision measurement and a dioptric power of 0.50D SPH is additionally required to satisfy a corrected vision of 1.0 as a result of the near vision measurement. Because a dioptric power of +0.50D SPH is additionally required as a result of the near vision measurement, it may be determined that the myopia is overcorrected to affect the near vision. Thus, in this case, the far vision is remeasured with a lens having a dioptric power of left eye -1.75D SPH, right eye -0.75D SPH, and -0.50D CYL@90° with a dioptric power of +0.50D SPH added thereto. As a result of the remeasurement, when there is no degradation in the far vision, because the overcorrection is removed, the remeasurement result may be updated as the far vision measurement result, and the corrected near vision may be improved when the overcorrection is removed.

As another example, it is assumed that the user is 45 years of age. When a lens having a dioptric power of left eye -1.50D SPH and right eye -1.50D SPH is prescribed for a corrected vision of 1.0 as a result of the far vision measurement and a dioptric power of +2.00D SPH is additionally required to satisfy a corrected vision of 1.0 as a result of the near vision measurement, it may be determined that the user is presbyopic. In this case, an additional prescription may be required to improve the corrected near vision. When a virtual image or a real object is located at a distance of less than 1 m, an extra focal power may be applied for focusing. The extra focal power may be determined as (1/distance from object [m]) - (residual accommodative power [D]). In a case where the near vision measurement is performed at a distance of 33 cm, because 33 cm is +3D, the user's residual accommodative power may be +1.00D equal to +3D minus an additional focal power +2D and an extra focal power required to focus on an object 75 cm away from the user may be determined as 1/0.75-1=0.33D.

In general, the additional dioptric power due to presbyopia may tend to be greater than the additional dioptric power due to overcorrection.

In operation S904, the vision correcting apparatus 1000 may control the focus adjustable lens based on the third dioptric power.

According to an embodiment of the disclosure, the dioptric power of the focus adjustable lens 1350 may be changed by an external stimulus or device setting, and particularly, in the case of a liquid crystal (LC) lens, the dioptric power may be adjusted by adjusting a voltage applied to each electrode according to an electrode arrangement for an LC element. Thus, the vision correcting apparatus 1000 may determine a voltage applied to the focus adjustable lens 1350 and control the focus adjustable lens 1350 by applying the determined voltage to the focus adjustable lens 1350.

According to an embodiment of the disclosure, when it is determined that the user is presbyopic, the vision correcting apparatus 1000 may differently control the dioptric power of the focus adjustable lens 1350 in a case where the user views a far object and in a case where the user views a near object, based on the output of the depth sensor or the eye gaze detecting module 1500, in order to suitably control a variable lens according to the dioptric power for far vision correction and the dioptric power for near vision correction.

According to an embodiment of the disclosure, when it is determined that the user is presbyopic, the vision correcting apparatus 1000 may control the focus adjustable lens 1350 such that a dioptric power for far vision correction may be applied to a first area and a dioptric power for near vision correction may be applied to a second area, by distinguishing between the areas of the focus adjustable lens 1350.

According to an embodiment of the disclosure, the vision correcting apparatus 1000 may include an augmented reality device, and the vision correcting apparatus 1000 may display a vision measurement table for vision measurement on a display of the augmented reality device and may perform far vision measurement and near vision measurement based on the output of the eye gaze detecting module 1500 or the user's response. A particular method of performing far vision measurement and near vision measurement by using the display of the augmented reality device will be described below with reference to FIGS. 10A to 11B.

FIG. 10A illustrates a method of measuring far vision in a vision correcting method according to an embodiment of the disclosure.

Referring to FIG. 10A, a vision measurement table may be displayed at a distance of about 5 m to about 6 m and far vision may be measured based on a user response as to whether a letter corresponding to each focal power (vision) is well seen. When the user's far vision is measured by using the vision correcting apparatus 1000 according to an embodiment of the disclosure, the display unit may project and display a vision measurement table image at an infinite distance. The vision correcting apparatus 1000 according to an embodiment of the disclosuremay project and display a character with a size corresponding to each focal power (vision) at a distance of about 5 m to about 6 m and may measure the vision by a subjective refraction test (subjective refractive) method of obtaining a response as to whether the character is clearly seen. In this case, displaying an image at an infinite distance by a vision measuring device may represent the same meaning as displaying an image at a distance of about 5 m to about 6 m.

In the vision correcting method according to an embodiment of the disclosure, the vision correcting apparatus 1000 may use a fogging method for artificially suppressing the accommodative power in order to measure the vision of ametropia (myopia, hyperopia, or astigmatism). The fogging method may be a representative subjective refraction test (subjective refractive) method and may be used to form an image "before" the retina by providing a lens with a suitable dioptric power (e.g., +5.0D). As such, when an accommodation effort is made in an artificially induced hyperopic state, because a focus is formed in further front of the retina and thus an image is more unclearly seen, the ciliary muscle may become an accommodation-relaxed state. After allowing enough time for the accommodation to be relaxed, a subjective refraction test may be performed while gradually reducing the dioptric power of a fogging lens at certain intervals (e.g., 0.25D).

In the fogging method, in order to determine the circle of least confusion, the dioptric power may be reduced until focusing on a virtual vision measurement table (virtual vision chart). In other words, a minus spherical dioptric power (SPH dioptric power) may be applied to the focus adjustable lens. In this case, the circle of least confusion may refer to the minimum circle in which the light passing through the lens is recognized as a single point by the human eyes. As such, when the vision is measured by using the fogging method, hyperopia undercorrection may be prevented.

For astigmatism correction, a cylindrical dioptric power (CYL dioptric power) may be applied. For example, when +0.25 SPH and -0.5 CYL are applied to both the x axis and the y axis and the vision is improved compared to the previous vision correction, CYL may be increased until the best vision is obtained.

When the user is myopic, a lens with a dioptric power having a minus diopter may be prescribed based on the result of measurement of the user's far vision. When the vision is self-measured without expert intervention, myopia overcorrection may occur. That is, as a result of the far vision measurement, a lens having a stronger focal power than an actually necessary focal power may be prescribed for correcting the user's myopia.

In the case of self-measuring the far vision, the main causes for overcorrection may be as follows.

First, it may be difficult for the vision correcting apparatus 1000 to clearly display a letter of a certain focal power or less for measuring the vision. This may be due to a resolution limit caused by the hardware performance of the vision correcting apparatus 1000. For example, it is assumed that the user's far vision is -3.0D and it is difficult for the vision correcting apparatus 1000 to clearly display a letter with a size corresponding to -3.5D or higher. When a letter with a size corresponding to -3.5D is displayed, the user may respond that the displayed letter is not well seen; however, this may be not because the user's vision is lower than -3.5D but because the resolution of the displayed letter is low. For example, it is assumed that a letter with the minimum size that may be output by a display device has a visual angle of 25' and the user's far vision is -3.0D. The letter with a visual angle of 25' may be distinguishable at a vision of 0.2 or higher, and it may be difficult for the user to sense a visual angle change due to a dioptric power change in a correction state of a vision of 0.2 or higher. The user with a far vision of -3.0D may respond that the letter is sufficiently well seen even when the vision correcting apparatus 1000 is undercorrected to -1.0D. However, this may be not because the user's vision is corrected but because the resolution of the displayed letter is low.

Second, when the user's vision is overcorrected by using a lens with a higher focal power than a lens with a focal power suitable for the user's actual vision, because the user may well see the letter in a vision test table, the user may respond the overcorrected vision as his/her own vision (or focal power). In order to more accurately measure the vision, the intervention of the accommodative power should be minimized, and in the case of excessive accommodation, myopia may be overcorrected because an image is formed before the retina. Particularly, it may be difficult to accurately determine the degree of accommodation when the user of the vision correcting apparatus 1000 self-measures the vision without the help of an expert.

When the vision is overcorrected, because a lens having a higher focal power than the user's vision is used, the user's eyes may be easily tired and the user's near vision may be affected. Particularly, overcorrection may be likely to occur in the vision test result of young myopic users.

The vision measuring and correcting method according to an embodiment of the disclosuremay prevent the overcorrection of myopia by using the near vision measurement result. In this case, the vision correcting apparatus 1000 according to an embodiment of the disclosuremay determine whether the user is presbyopic and the degree of presbyopia and may correct the overcorrection of myopia based on the far vision measurement result based on the determination result.

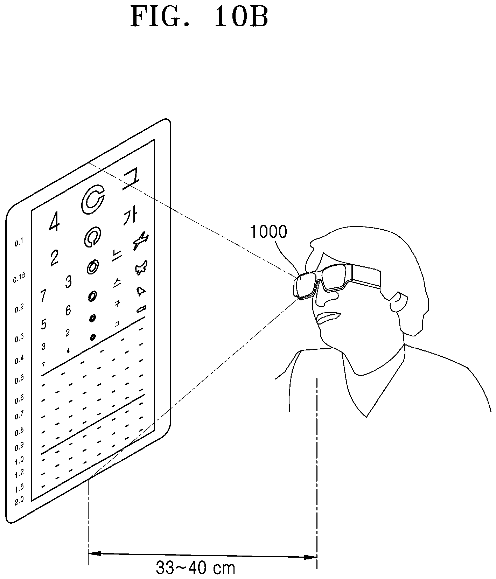

FIG. 10B illustrates a method of measuring near vision in a vision correcting method according to an embodiment of the disclosure.

The vision correcting apparatus 1000 according to an embodiment of the disclosure may perform near vision measurement after applying the focal power of the focus adjustable lens based on the far vision measurement result. For example, when the user is myopic, the focal power of the focus adjustable lens may be determined as a minus diopter, and when the user is not myopic, a separate focal power may not be applied to the focus adjustable lens.

According to an embodiment of the disclosure, because the light passing from a distance through the focus adjustable lens with the first dioptric power applied thereto forms an image on the retina, the user viewing a far object through the focus adjustable lens with the first dioptric power applied thereto may accurately recognize an image of the far object. However, when the user is presbyopic or the first dioptric power is excessively determined compared to the degree of myopia of the user, the focus adjustable lens with the first dioptric power applied thereto may not match the focus of light reflected from a near object and incident on the eye to the retina. Thus, the user's corrected near vision measured based on the focus adjustable lens 1350 with the first dioptric power applied thereto may require an additional dioptric power adjustment, that is, the second dioptric power, to match near focus.

The vision correcting apparatus 1000 according to an embodiment of the disclosure may measure corrected near vision. In this case, the measurement of the corrected near vision may be performed while the user is wearing the focus adjustable lens to which the first dioptric power determined based on the far vision has been applied.

Referring to FIG. 10B, a vision measurement table may be displayed at a distance of about 33 cm to about 40cm and near vision may be measured based on a user response as to whether a letter corresponding to each focal power (vision) is well seen.

When the user's near vision is measured by using the vision correcting apparatus 1000 according to an embodiment of the disclosure, the display unit may project and display a vision measurement table image at a distance of about 33 cm to about 40 cm from the user's eyes.

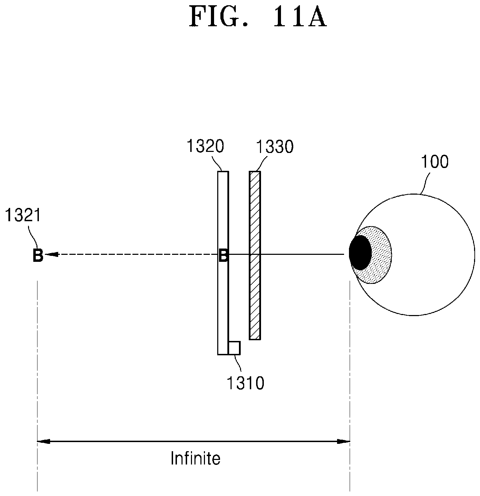

Because the vision correcting apparatus 1000 using a waveguide display provides a virtual image on a single focal plane, the user's eyes may perceive that an image is projected at an infinite distance due to vergence-accommodation conflict (VAC). However, because two displays having complementary diopters are additionally arranged on both sides of the waveguide display in the vision correcting apparatus 1000 according to an embodiment of the disclosure, the user may perceive that an image is projected at a short distance. A particular method of additionally arranging a display for near projection will be described below with reference to FIGS. 11A and 11B.

In the vision correcting method according to an embodiment of the disclosure, in order to measure the near vision, the vision correcting apparatus 1000 may gradually (e.g., +0.25D SPH) increase the focal power until the user may read the vision measurement table at a short distance. In this case, the finally determined extra focal power may be the degree of myopia overcorrection or the focal power of a lens for presbyopic magnifying glasses. Even when the user is myopic, when the user is also presbyopic, a lens with a dioptric power having a minus diopter for far focus adjustment and a lens with a dioptric power having a plus diopter for near focus adjustment may be prescribed together.

According to an embodiment of the disclosure, when the user is of a certain threshold age or more and a lens with a dioptric power having a plus diopter is additionally required in order to focus on a near object as a result of measuring the user's near vision to which a myopia correcting lens determined based on the result of the far vision measurement has been applied, the vision correcting apparatus 1000 may determine that the user is presbyopic. On the other hand, when the user is of less than the certain threshold age and a lens with a dioptric power having a plus diopter is additionally required in order to focus on a near object as a result of measuring the user's near vision to which a myopia correcting lens determined based on the result of the far vision measurement has been applied, the vision correcting apparatus 1000 may determine that the myopia correcting lens determined based on the result of the user's far vision measurement is overcorrected.

According to an embodiment of the disclosure, the vision correcting apparatus 1000 may obtain at least one of the far vision measurement result or the near vision measurement result of the user through the user input unit. For example, when the user knows the vision measurement result measured at the hospital or the like, the user may input the vision measurement result through the user input unit 1100 or the microphone 1200, and the vision correcting apparatus 1000 may determine the focal power (dioptric power) of the focus adjustable lens based on the vision measurement result obtained through the user input.

According to an embodiment of the disclosure, when the user measures the vision by using a real (physical) vision measurement table, the vision correcting apparatus 1000 may determine whether the distance is suitable for vision measurement, based on the distance between the user and the vision measurement table by using the depth sensor. Based on the result of determining the distance between the user and the vision measurement table, the vision correcting apparatus 1000 may output a voice for instructing the user to move toward or away from the vision measurement table through the speaker or may output a phrase for instructing the user to move toward or away from the vision measurement table through the display.

FIGS. 11A and 11B are diagrams for describing a method of measuring near vision by using a vision correcting apparatus in a vision correcting method according to an embodiment of the disclosure.

It is assumed that the optical engine 1310 generates a virtual image B 1321 and displays the virtual image B 1321 on a see-through display 1320.

Referring to FIG. 11A, the vision correcting apparatus 1000 according to an embodiment of the disclosure may include a see-through display 1320 and a focus adjustable lens 1330 for vision correction. The virtual image B 1321 displayed on the see-through display 1320 may be perceived as being projected at an infinite distance, and the human eyes may recognize the virtual image B 1321 as a far object. Thus, when the see-through display 1320 is used, a separate process for perceiving that the virtual image B is projected at a short distance from the user's eyes may be required for measurement of the near vision.

Referring to FIG. 11B, the vision correcting apparatus 1000 according to an embodiment of the disclosure may include a second focus adjustable lens 1332 having a minus diopter and a third focus adjustable lens 1333 having a plus diopter, in addition to a see-through display 1320 and a first focus adjustable lens 1331 for vision correction. In this case, the diopters of the second focus adjustable lens 1332 and the third focus adjustable lens 1333 may have the same absolute value and may be arranged on both sides of the see-through display 1320.

The second focus adjustable lens 1332 may pull a virtual image displayed on the see-through display 1320 to be recognized at a short distance, and the third focus adjustable lens 1333 may compensate for the influence of the second focus adjustable lens 1332 to focus on a real object (real world) the user is looking at.

A real object A 200 seen through the see-through display 1320 may be recognized by the user's eye 100 through the third focus adjustable lens 1333 having a plus diopter and the second focus adjustable lens 1332 having a minus diopter. In this case, because the diopters of the second focus adjustable lens 1332 and the third focus adjustable lens 1333 have the same absolute value, the light passing through both the second focus adjustable lens and the third focus adjustable lens may not be refracted.

A virtual image B 1321 displayed on the see-through display 1320 may be recognized by the user's eye through only the second focus adjustable lens having a minus diopter. Because the second focus adjustable lens 1332 has a dioptric power of a minus diopter (e.g., -3.0D), the virtual image B 1321 displayed on the see-through display 1320 may be refracted by the second focus adjustable lens 1332 and accordingly the image position thereof may be pulled. Thus, the vision correcting apparatus 1000 according to an embodiment of the disclosure may measure the user's near vision by providing a vision measurement table for vision measurement at a short distance (about 33 cm to about 40 cm).

The vision correcting apparatus 1000 according to an embodiment of the disclosure may be implemented in a form in which the first focus adjustable lens 1331 and the second focus adjustable lens 1332 are merged into one focus adjustable lens. In this case, the merged focus adjustable lens may have the same dioptric power as the dioptric power obtained by synthesizing the dioptric powers of the first focus adjustable lens 1331 and the second focus adjustable lens 1332.

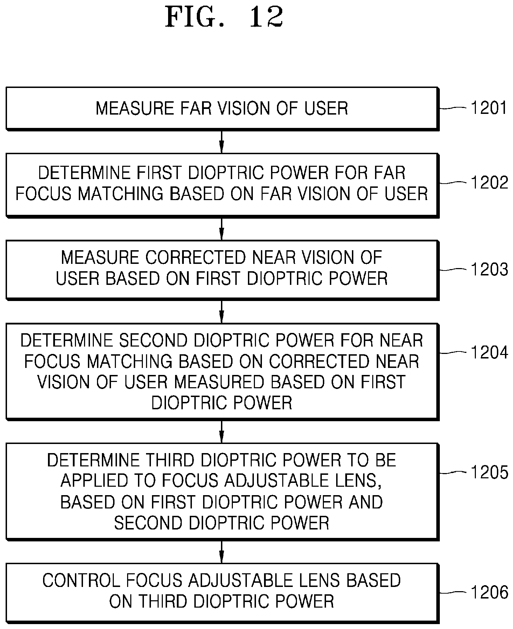

FIG. 12 is a flowchart illustrating a vision correcting method according to an embodiment of the disclosure.

Referring to FIG. 12, the vision correcting apparatus 1000 according to an embodiment of the disclosure may measure the user's vision and correct the user's vision based on the measured vision. In FIG. 12, redundant descriptions with those of FIGS. 9 to 11B may be omitted or briefly described.

In operation 1201, the vision correcting apparatus 1000 may measure the user's far vision.

The vision measurement table may be displayed at a distance of about 5 m to about 6 m and the far vision may be measured based on a user response as to whether a letter corresponding to each focal power (vision) is well seen. When the user's far vision is measured by using the vision correcting apparatus 1000 according to an embodiment of the disclosure, the display unit may project and display a vision measurement table image at an infinite distance. The vision correcting apparatus 1000 according to an embodiment of the disclosure may project and display a character with a size corresponding to each focal power (vision) at a distance of about 5 m to about 6 m and may measure the vision by a subjective refraction test (subjective refractive) method of obtaining a response as to whether the character is clearly seen. In this case, displaying an image at an infinite distance by a vision measuring device may represent the same meaning as displaying an image at a distance of about 5 m to about 6 m.

In operation 1202, the vision correcting apparatus 1000 may determine a first dioptric power to match far focus based on the user's far vision.

When the user is myopic, a lens with a first dioptric power having a minus diopter may be prescribed based on the result of measurement of the user's far vision.

In operation 1203, the vision correcting apparatus 1000 may measure the user's corrected near vision based on the first dioptric power.

When the vision is self-measured by using a subjective refraction test method without expert intervention, myopia overcorrection may occur. When myopia is overcorrected, additional refractive control may be required to correct the user's near vision. Thus, the vision correcting apparatus according to an embodiment of the disclosure may measure the user's corrected near vision in order to identify whether overcorrection occurs. In this case, the measurement of the corrected near vision may be performed while the user is wearing the focus adjustable lens to which the first dioptric power determined based on the far vision has been applied.

The vision measurement table may be displayed at a distance of about 33 cm to about 40 cm and the near vision may be measured based on a user response as to whether a letter corresponding to each focal power (vision) is well seen. When the user's near vision is measured by using the vision correcting apparatus 1000 according to an embodiment of the disclosure, the display unit may project and display a vision measurement table image at a distance of about 33 cm to about 40 cm from the user's eyes.

Because the vision correcting apparatus 1000 using a waveguide display provides a virtual image on a single focal plane, the user's eyes may perceive that an image is projected at an infinite distance [due to VAC]; however, because two displays having complementary diopters are additionally arranged on both sides of the waveguide display in the vision correcting apparatus 1000 according to an embodiment of the disclosure, the user may perceive that an image is projected at a short distance.

In operation 1204, the vision correcting apparatus 1000 may determine a second dioptric power to match near focus based on the user's corrected near vision measured based on the first dioptric power.

When the user is presbyopic or the first dioptric power is excessively determined compared to the degree of myopia of the user, the focus adjustable lens with the first dioptric power applied thereto may not match the focus of light reflected from a near object and incident on the eye to the retina. Thus, the user's corrected near vision measured based on the focus adjustable lens 1350 with the first dioptric power applied thereto may require an additional dioptric power adjustment, that is, the second dioptric power, to match near focus. In this case, in the case of presbyopia or myopia overcorrection, because the near focus of light passing through the eye lens is formed behind the retina, the second dioptric power may have a positive value and the magnitude of the second dioptric power may vary according to the degree of presbyopia of the user or the degree of myopia overcorrection. Because a lens having a positive dioptric power (e.g., a convex lens) concentrates the light incident on the lens, when a positive dioptric power is applied to the focus adjustable lens 1350, the near focal length of light passing through the eye lens may decrease and thus a near image may be formed on the retina.

**In** operation 1205, the vision correcting apparatus 1000 may determine a third dioptric power to be applied to the focus adjustable lens, based on the first dioptric power and the second dioptric power.

According to an embodiment of the disclosure, when it is determined that the user is not presbyopic, the third dioptric power may be determined as the sum of the first dioptric power and the second dioptric power.

According to an embodiment of the disclosure, when it is determined that the user is presbyopic, the third dioptric power may include a plurality of dioptric powers including the first dioptric power to match far focus and a fourth dioptric power to match near focus, and the fourth dioptric power may refer to a dioptric power to match near focus that is determined based on the residual accommodative power of the user's eyes and the second dioptric power.

In operation 1206, the vision correcting apparatus 1000 may control the focus adjustable lens based on the third dioptric power.

According to an embodiment of the disclosure, the dioptric power of the focus adjustable lens 1350 may be changed by an external stimulus or device setting, and particularly, in the case of a liquid crystal (LC) lens, the dioptric power may be adjusted by adjusting a voltage applied to each electrode according to an electrode arrangement for an LC element. Thus, the vision correcting apparatus 1000 may determine a voltage applied to the focus adjustable lens 1350 and control the focus adjustable lens 1350 by applying the determined voltage to the focus adjustable lens 1350.

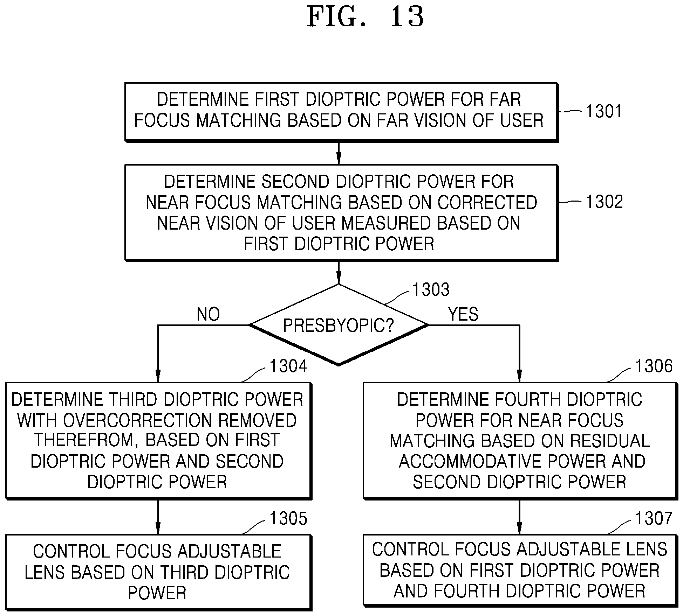

FIG. 13 is a flowchart illustrating a vision correcting method according to an embodiment of the disclosure.

Referring to FIG. 13, the vision correcting apparatus 1000 according to an embodiment of the disclosure may determine whether the user is presbyopic, based on the user's far vision and corrected near vision, and correct the user's vision based on whether the user is presbyopic. In FIG. 13, redundant descriptions with those of FIGS. 9 to 12 may be omitted or briefly described.

In operation 1301, the vision correcting apparatus 1000 may determine a first dioptric power to match far focus based on the user's far vision.

When the user is myopic, a lens with a first dioptric power having a minus diopter may be prescribed based on the result of measurement of the user's far vision.

In operation 1302, the vision correcting apparatus 1000 may determine a second dioptric power to match near focus based on the user's corrected near vision measured based on the first dioptric power.

When the user is presbyopic or the first dioptric power is excessively determined compared to the degree of myopia of the user, the focus adjustable lens with the first dioptric power applied thereto may not match the focus of light reflected from a near object and incident on the eye to the retina. Thus, the user's corrected near vision measured based on the focus adjustable lens 1350 with the first dioptric power applied thereto may require an additional dioptric power adjustment, that is, the second dioptric power, to match near focus.

In operation 1303, the vision correcting apparatus 1000 may identify whether the user is presbyopic.

The vision measuring and correcting method according to an embodiment of the disclosure may identify whether the user is presbyopic or the degree of presbyopia based on the user's age information or the magnitude of the second dioptric power. In general, the magnitude of the second dioptric power due to presbyopia may tend to be greater than the additional dioptric power due to overcorrection.

In operation 1304, when it is determined that the user is not presbyopic as a result of the identification in operation 1303, the vision correcting apparatus 1000 may determine a third dioptric power to be applied to the focus adjustable lens, based on the first dioptric power and the second dioptric power.

According to an embodiment of the disclosure, because it is determined that the user is not presbyopic, the third dioptric power may be determined as the sum of the first dioptric power and the second dioptric power. In this case, a dioptric power for correcting the user's myopia may be determined as the third dioptric power.

In operation 1305, the vision correcting apparatus 1000 may control the focus adjustable lens based on the third dioptric power.

According to an embodiment of the disclosure, the dioptric power of the focus adjustable lens 1350 may be changed by an external stimulus or device setting, and particularly, in the case of a liquid crystal (LC) lens, the dioptric power may be adjusted by adjusting a voltage applied to each electrode according to an electrode arrangement for an LC element. Thus, the vision correcting apparatus 1000 may determine a voltage applied to the focus adjustable lens 1350 and control the focus adjustable lens 1350 by applying the determined voltage to the focus adjustable lens 1350.

In operation 1306, when it is determined that the user is presbyopic as a result of the identification in operation 1303, the vision correcting apparatus 1000 may determine a fourth dioptric power to match near focus based on the residual accommodative power and the second dioptric power.

According to an embodiment of the disclosure, when it is determined that the user is presbyopic, the third dioptric power may include a plurality of dioptric powers including the first dioptric power to match far focus and a fourth dioptric power to match near focus, and the fourth dioptric power may refer to a dioptric power to match near focus that is determined based on the residual accommodative power of the user's eyes and the second dioptric power.

According to an embodiment of the disclosure, the fourth dioptric power may be determined as (1/distance from object [m]) - (residual accommodative power [D]). For example, the extra focal power required to focus on an object 75 cm away from the user with a residual accommodative power of +1.00D may be determined as 1/0.75-1=0.33D.

In operation 1307, the vision correcting apparatus 1000 may control the focus adjustable lens based on the first dioptric power and the fourth dioptric power.

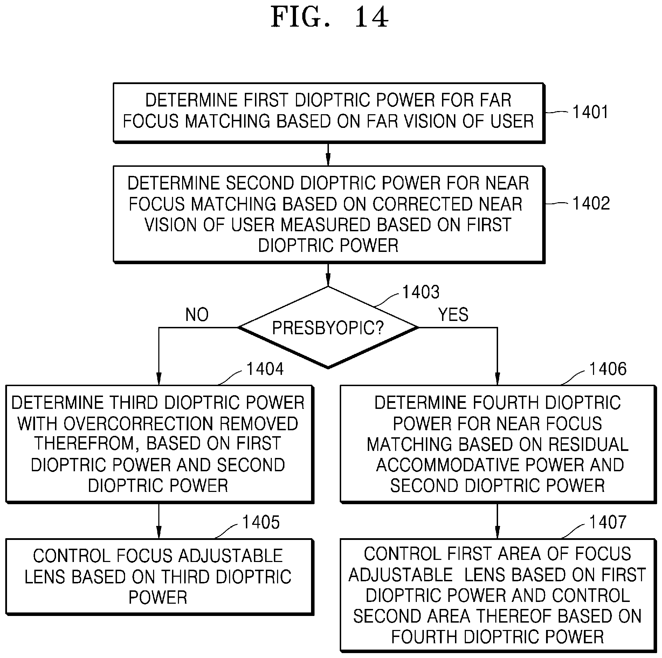

FIG. 14 is a flowchart illustrating a vision correcting method according to an embodiment of the disclosure.

Referring to FIG. 14, the vision correcting apparatus 1000 according to an embodiment of the disclosure may determine whether the user is presbyopic, based on the user's far vision and corrected near vision, and apply different dioptric powers for the areas of the focus adjustable lens when the user is presbyopic. In FIG. 14, redundant descriptions with those of FIGS. 9 to 13 may be omitted or briefly described.

In operation 1401, the vision correcting apparatus 1000 may determine a first dioptric power to match far focus based on the user's far vision.

When the user is myopic, a lens with a first dioptric power having a minus diopter may be prescribed based on the result of measurement of the user's far vision.

In operation 1402, the vision correcting apparatus 1000 may determine a second dioptric power to match near focus based on the user's corrected near vision measured based on the first dioptric power.

In operation 1403, the vision correcting apparatus 1000 may identify whether the user is presbyopic.

In operation 1404, when it is determined that the user is not presbyopic as a result of the identification in operation 1403, the vision correcting apparatus 1000 may determine a third dioptric power to be applied to the focus adjustable lens, based on the first dioptric power and the second dioptric power.

In operation 1405, the vision correcting apparatus 1000 may control the focus adjustable lens based on the third dioptric power.

According to an embodiment of the disclosure, the dioptric power of the focus adjustable lens 1350 may be changed by an external stimulus or device setting, and particularly, in the case of a liquid crystal (LC) lens, the dioptric power may be adjusted by adjusting a voltage applied to each electrode according to an electrode arrangement for an LC element. Thus, the vision correcting apparatus 1000 may determine a voltage applied to the focus adjustable lens 1350 and control the focus adjustable lens 1350 by applying the determined voltage to the focus adjustable lens 1350.

In operation 1406, when it is determined that the user is presbyopic as a result of the identification in operation 1403, the vision correcting apparatus 1000 may determine a fourth dioptric power to match near focus based on the residual accommodative power and the second dioptric power.

According to an embodiment of the disclosure, the fourth dioptric power may be determined as (1/distance from object [m]) - (residual accommodative power [D]). For example, the extra focal power required to focus on an object 75 cm away from the user with a residual accommodative power of +1.00D may be determined as 1/0.75-1=0.33D.

In operation 1407, the vision correcting apparatus 1000 may control the first area of the focus adjustable lens based on the first dioptric power and control the second area thereof based on the fourth dioptric power.

According to an embodiment of the disclosure, when it is determined that the user is presbyopic, the third dioptric power may include a plurality of dioptric powers including the first dioptric power to match far focus and a fourth dioptric power to match near focus, and the fourth dioptric power may refer to a dioptric power to match near focus that is determined based on the residual accommodative power of the user's eyes and the second dioptric power.

In the vision correcting apparatus 1000 according to an embodiment of the disclosure, the focus adjustable lens 1350 may differently adjust the dioptric power of an element corresponding to an electrode by applying a different voltage to each electrode. Thus, the focus adjustable lens may be divided into a plurality of areas, the first area may be controlled based on the first dioptric power in order to correct the far vision, and the second area may be controlled based on the fourth dioptric power in order to correct the near vision.

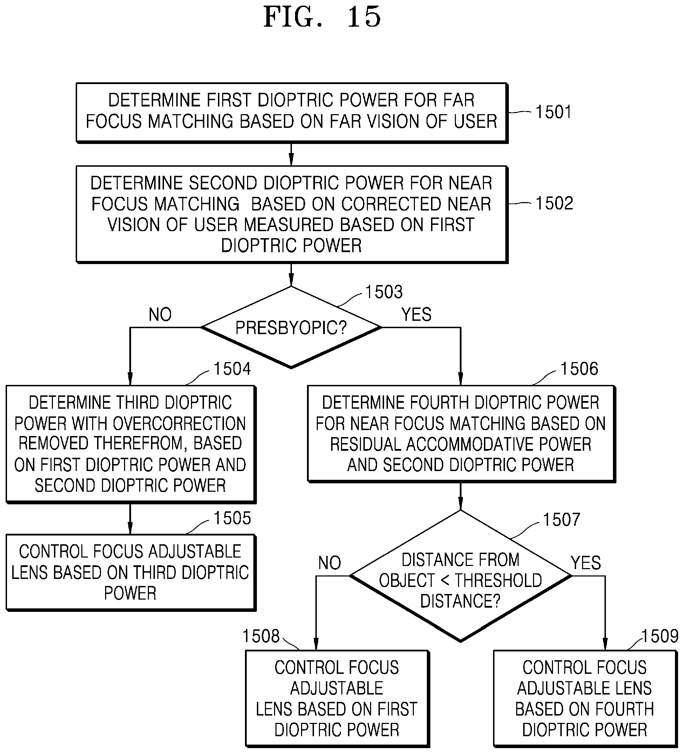

FIG. 15 is a flowchart illustrating a vision correcting method according to an embodiment of the disclosure.

Referring to FIG. 15, the vision correcting apparatus 1000 according to an embodiment of the disclosuremay determine whether the user is presbyopic, based on the user's far vision and corrected near vision, and determine the dioptric power based on the distance from an object when the user is presbyopic. In FIG. 15, redundant descriptions with those of FIGS. 9 to 14 may be omitted or briefly described.

In operation 1501, the vision correcting apparatus 1000 may determine a first dioptric power to match far focus based on the user's far vision.

When the user is myopic, a lens with a first dioptric power having a minus diopter may be prescribed based on the result of measurement of the user's far vision.

In operation 1502, the vision correcting apparatus 1000 may determine a second dioptric power to match near focus based on the user's corrected near vision measured based on the first dioptric power.

In operation 1503, the vision correcting apparatus 1000 may identify whether the user is presbyopic.

In operation 1504, when it is determined that the user is not presbyopic as a result of the identification in operation 1503, the vision correcting apparatus 1000 may determine a third dioptric power to be applied to the focus adjustable lens, based on the first dioptric power and the second dioptric power.

In operation 1505, the vision correcting apparatus 1000 may control the focus adjustable lens based on the third dioptric power.

In operation 1506, when it is determined that the user is presbyopic as a result of the identification in operation 1503, the vision correcting apparatus 1000 may determine a fourth dioptric power to match near focus based on the residual accommodative power and the second dioptric power.

According to an embodiment of the disclosure, when it is determined that the user is presbyopic, the third dioptric power may include a plurality of dioptric powers including the first dioptric power to match far focus and the fourth dioptric power to match near focus.

In operation 1507, the vision correcting apparatus 1000 may identify whether the distance from the object is less than a threshold distance.

According to an embodiment of the disclosure, the vision correcting apparatus 1000 may measure the distance from an object located in front of the vision correcting apparatus 1000 based on the output of the depth sensor, may correct the far vision when the measured distance is greater than a certain threshold distance, and may correct the near vision when the measured distance is less than a certain threshold distance. In this case, the certain threshold distance may be determined based on the user's near vision.

According to an embodiment of the disclosure, the vision correcting apparatus 1000 may measure the distance from an object located in front of the vision correcting apparatus 1000 based on the output of the depth sensor, may correct the user's far vision when the measured distance is greater than a first threshold distance (e.g., 4 m), and may correct the user's near vision when the measured distance is less than a second threshold distance (e.g., 20 cm). In this case, the first threshold distance and the second threshold distance may be determined based on the user's far vision and near vision.

According to an embodiment of the disclosure, the vision correcting apparatus 1000 may identify the user's eye gaze direction based on the result of tracking the user's eye gaze by using the eye gaze tracking sensor 1510, may measure the distance from an object located in the identified user's eye gaze direction, and may identify whether the distance from the object is less than a threshold distance.

In operation 1508, the vision correcting apparatus 1000 may control the focus adjustable lens based on the first dioptric power.

When it is identified in operation 1507 that the distance from the object is not less than the threshold distance, the vision correcting apparatus 1000 may control the focus adjustable lens 1350 based on the first dioptric power in order to correct the far vision.

In operation 1509, the vision correcting apparatus 1000 may control the focus adjustable lens based on the fourth dioptric power.

When it is identified in operation 1507 that the distance from the object is less than the threshold distance, the vision correcting apparatus 1000 may control the focus adjustable lens 1350 based on the fourth dioptric power in order to correct the near vision.

The machine-readable storage medium may be provided in the form of a non-transitory storage medium. Here, the term "non-transitory storage medium" may mean that the storage medium is a tangible device and does not include signals (e.g., electromagnetic waves), and may mean that data may be semipermanently or temporarily stored in the storage medium. For example, the "non-transitory storage medium" may include a buffer in which data is temporarily stored.

According to an embodiment of the disclosure, the method according to various embodiments described herein may be included and provided in a computer program product. The computer program product may be traded as a product between a seller and a buyer. The computer program product may be distributed in the form of a machine-readable storage medium (e.g., a compact disc read only memory (CD-ROM)) or may be distributed (e.g., downloaded or uploaded) online through an application store or directly between two user devices (e.g., smartphones). In the case of online distribution, at least a portion of the computer program product (e.g., a downloadable app) may be at least temporarily stored or temporarily generated in a machine-readable storage medium such as a memory of a manufacturer server, a memory of an application store server, or a memory of a relay server.

## Claims

1. A method of correcting vision by a vision correcting apparatus comprising a focus adjustable lens, the method comprising:
determining a first dioptric power for matching far focus, based on far vision of a user;
determining a second dioptric power for matching near focus, based on corrected near vision of the user measured based on the first dioptric power;
determining at least one third dioptric power to be applied to the focus adjustable lens, based on the first dioptric power and the second dioptric power; and
controlling the focus adjustable lens based on the at least one third dioptric power.

2. The method of claim 1, wherein the determining the at least one third dioptric power comprises:
determining whether the user is presbyopic;
based on determining that the user is not presbyopic, determining the at least one third dioptric power by removing overcorrection of the first dioptric power based on the second dioptric power; and
based on determining that the user is presbyopic, determining a fourth dioptric power to correct near vision of the user and the first dioptric power as the at least one third dioptric power.

3. The method of claim 1, further comprising:
measuring the far vision of the user; and
measuring the corrected near vision of the user based on the first dioptric power.

4. The method of claim 1, wherein the corrected near vision of the user is near vision of the user measured based on the focus adjustable lens with the first dioptric power applied to the focus adjustable lens.

5. The method of claim 2, wherein the controlling the focus adjustable lens based on the at least one third dioptric power comprises:
controlling a first area of the focus adjustable lens based on the first dioptric power; and
controlling a second area of the focus adjustable lens based on the fourth dioptric power.

6. The method of claim 2, wherein the controlling the focus adjustable lens based on the at least one third dioptric power comprises:
identifying a distance between the vision correcting apparatus and an object;
determining whether the distance between the vision correcting apparatus and the object is less than a threshold distance;
based on determining that the distance between the vision correcting apparatus and the object is less than the threshold distance, controlling the focus adjustable lens based on the first dioptric power; and
based on determining that the distance between the vision correcting apparatus and the object is greater than or equal to the threshold distance, controlling the focus adjustable lens based on the fourth dioptric power.

7. The method of claim 6, wherein the controlling the focus adjustable lens based on the at least one third dioptric power further comprises obtaining eye gaze information of the user, and
wherein the identifying the distance between the vision correcting apparatus and the object comprises identifying the distance between the vision correcting apparatus and the object based on the eye gaze information of the user.

8. An apparatus for correcting vision, the apparatus comprising:
a focus adjustable lens;
a storage storing a program comprising at least one instruction; and
at least one processor configured to execute the at least one instruction stored in the storage unit,
wherein the at least one processor is configured to execute the at least one instruction to:
determine a first dioptric power for matching far focus, based on far vision of a user,
determine a second dioptric power for matching near focus based on corrected near vision of the user measured, based on the first dioptric power,
determine at least one third dioptric power to be applied to the focus adjustable lens, based on the first dioptric power and the second dioptric power, and
control the focus adjustable lens based on the at least one third dioptric power.

9. The apparatus of claim 8, wherein the at least one processor is further configured to execute the at least one instruction to:
determine whether the user is presbyopic,
based on determining that the user is not presbyopic, determine the at least one third dioptric power by removing overcorrection of the first dioptric power based on the second dioptric power, and
based on determining that the user is presbyopic, determine a fourth dioptric power to correct near vision of the user and the first dioptric power as the at least one third dioptric power.

10. The apparatus of claim 8, wherein the at least one processor is further configured to execute the at least one instruction to:
measure the far vision of the user, and
measure the corrected near vision of the user based on the first dioptric power.

11. The apparatus of claim 8, wherein the corrected near vision of the user is near vision of the user measured based on the focus adjustable lens with the first dioptric power applied to the focus adjustable lens.

12. The apparatus of claim 9, wherein the at least one processor is further configured to execute the at least one instruction to control a first area of the focus adjustable lens based on the first dioptric power and control a second area of the focus adjustable lens based on the fourth dioptric power.

13. The apparatus of claim 9, wherein the at least one processor is further configured to execute the at least one instruction to:
identify a distance between a vision correcting apparatus and an object,
determine whether the distance between the vision correcting apparatus and the object is less than a threshold distance,
based on determining that the distance between the vision correcting apparatus and the object is less than the threshold distance, control the focus adjustable lens based on the first dioptric power, and
based on determining that the distance between the vision correcting apparatus and the object is greater than or equal to the threshold distance, control the focus adjustable lens based on the fourth dioptric power.

14. The apparatus of claim 13, wherein the at least one processor is further configured to execute the at least one instruction to:
obtain eye gaze information of the user, and
identify the distance between the vision correcting apparatus and the object based on the eye gaze information of the user.

15. A non-transitory computer-readable recording medium has recorded thereon a program that is executable by a processor of a vision correcting apparatus to perform a method of any one of claims 1 to 7.
